# EUROPEAN PATENT SPECIFICATION

(11) **EP 4 077 713 B1**
(45) Date of publication and mention of the grant of the patent: **26.07.2023**
(21) Application number: 20829359.7
(22) Date of filing: 16.12.2020
(51) Int. Cl.: C12Q 1/6804, C12N 15/10

(54) **METHOD AND KIT FOR WHOLE GENOME AMPLIFICATION AND ANALYSIS OF TARGET MOLECULES IN A BIOLOGICAL SAMPLE**
VERFAHREN UND KIT ZUR GESAMTGENOMAMPLIFIKATION UND -ANALYSE VON ZIELMOLEKÜLEN IN EINER BIOLOGISCHEN PROBE.
PROCÉDÉ ET KIT POUR L'AMPLIFICATION ET L'ANALYSE DU GÉNOME ENTIER DE MOLÉCULES CIBLES DANS UN ÉCHANTILLON BIOLOGIQUE

(30) Priority: 16.12.2019 IT 201900024159
(43) Date of publication of application: 26.10.2022
(73) Proprietor: Menarini Silicon Biosystems S.p.A., 40013 Castel Maggiore (BO) (IT)
(72) Inventor: MANARESI, Nicolò, 40013 Castel Maggiore (BO) (IT); RASPADORI, Andrea, 40013 Castel Maggiore (BO) (IT); FERRARINI, Alberto, 40013 Castel Maggiore (BO) (IT)
(74) Representative: Studio Torta S.p.A.
(86) International application number: PCT/IB2020/062053
(87) International publication number: WO 2021/124166

(56) References cited:
- WO-A1-2018/144813
- US-A1- 2019 360 044

## Description

### Technical Field of the Invention

The present invention relates to a method and a kit for whole genome amplification and analysis of target molecules, in particular quantification of proteins, in a biological sample, in particular in a single cell sample.

### Prior Art

Methods for single-cell analysis allow to obtain information on the status of a cell without the complication resulting from heterogeneity in a bulk sample. Analysis of proteome and genome in the same single cell provides correlations between the cell's phenotype and its genotype, thus enabling unique insights into diverse biological and pathological processes. This is particularly true for tumors, in which somatically acquired genetic heterogeneity and its effect on transcription and protein translation are key components of the initiation and evolution of cancer.

Whole Genome Amplification (WGA) is useful to analyze the genome from single cells in order to obtain more DNA and simplify and/or allow different types of genetic analyses, including sequencing, SNP detection etc. WGA with a LM-PCR based on a Deterministic Restriction Site (DRS-WGA) is known from EP1109938.

WO 2017/178655 and WO 2019/016401 teach a simplified method to prepare massively parallel sequencing libraries from DRS-WGA (e.g. Ampli1^{™} WGA) or MALBAC for low-pass whole genome sequencing and copy number profiling.

Recently, methods for the simultaneous analysis of genome and transcriptome in single cells have been developed. The paper by Dey S. et al., 2015, Integrated genome and transcriptome sequencing of the same cell. Nature Biotechnology, 33(3), 285-289. http://doi.org/10.1038/nbt.3129, teaches a method by which messenger RNAs from single cells isolated by hand are first transcribed to single stranded cDNA and then amplified together with genomic DNA through a quasilinear whole-genome amplification. Two different libraries, one from cDNA and one from genomic DNA are then prepared and sequenced. In another approach, Macaulay, I. C., et al., 2015, G&T-seq: Parallel sequencing of single-cell genomes and transcriptomes. Nature Methods, 12(6), 519-522. http://doi.org/10.1038/nmeth.3370, mRNA is physically separated from gDNA using oligo-dT-coated beads to capture and isolate the polyadenylated mRNA molecules from a fully lysed single cell. The mRNA is then amplified using a modified Smart-seq2 protocol (Picelli, S. et al., 2013, Smart-seq2 for sensitive full-length transcriptome profiling in single cells. Nature Methods, 10(11), 1096-1100. http://doi.org/10.1038/nmeth.2639), while the gDNA can be amplified with available whole genome amplification methods and sequenced. These methods, while useful to link genotype to messenger transcription, do not allow to get a direct detection of proteins, the translation and turnover/degradation of which are actively regulated in the cell.

Currently, the most widely applied single-cell protein detection approaches rely on targeting specific proteins using tagged antibodies. Fluorescence-based detection and quantitation of proteins by fluorescence-activated cell sorting (FACS) or fluorescence microscopy allow protein detection in single cells with a low level of multiplexing by means of fluorescently labeled antibodies recognizing specific cell proteins. However this approach is generally limited to 10-15 simultaneous measurements as fluorophore-based highly multiplexed assays are challenged by spectral overlap between the emission spectra of multiple dyes. Moreover, complex algorithms are needed to deconvolute the overlapping spectra.

Fluidigm mass cytometer (CyTOF^{™}) employs metal-containing polymer tagged (MAXPAR^{™}) antibodies to detect proteins. The instrument is based on a non-optical physical principle of detection and a different chemical nature of labels. The fluorescent labels are replaced by specially designed multi-atom elemental tags and detection takes advantage of the high resolution, sensitivity, and speed of analysis of Time-of-Flight Mass Spectrometry (TOF-MS). Since many available stable isotopes can be used as tags, many proteins can potentially be detected simultaneously in individual cells [Ornatsky, O. et al, 2010, Highly multiparametric analysis by mass cytometry. Journal of Immunological Methods, 361(1-2), 1-20. http://doi.org/10.1016/j.jim.2010.07.002]. The work by Frei et al., 2016, Highly multiplexed simultaneous detection of RNAs and proteins in single cells. Nature Methods, 13(3), 269-275. http://doi.org/10.1038/nmeth.3742, teaches a method for simultaneous detection of RNAs and proteins in single cells based on Proximity Ligation Assay for RNA (PLAYR). PLAYR enables highly multiplexed quantification of transcripts in single cells by mass-cytometry enabling simultaneous quantification of more than 40 different mRNAs and proteins. Finally, mass cytometry allowed to investigate multiple cellular processes and phenotypic characteristics, along with proteins and messenger RNAs transcription, such as protein phosphorylation (Bendall, S. C. et. Al., 2011, Single-Cell Mass Cytometry of Differential Immune and Drug Responses Across a Human Hematopoietic Continuum. Science, 332(6030), 687-696. http://doi.org/10.1126/science.1198704) and cellular proliferation (Behbehani, G. K. et al., 2012, Single-cell mass cytometry adapted to measurements of the cell cycle. Cytometry Part A, 81A(7), 552-566. http://doi.org/10.1002/cyto.a.22075) .

The limitations of these approaches are that:
- due to the dynamics of ion flight in the mass spectrometer, the throughput of mass cytometry lags behind that of fluorescence-based instruments. Additionally, the sensitivity of mass reporters falls shy of few, more quantum-efficient fluorophores (such as phycoerythrin), making it harder to measure molecular features that are expressed at very low levels using mass cytometry (Spitzer, M. H. et al., 2016, Mass Cytometry: Single Cells, Many Features. Cell, 165(4), 780-791. http://doi.org/10.1016/j.cell.2016.04.019) .
- Importantly, because cells are atomized and ionized, cells cannot be recovered after the analysis and therefore genomic DNA cannot be analyzed.

Methods for the detection of proteins through oligonucleotide-labeled antibodies have been described in a paper by Fredriksson et al., 2002, Protein detection using proximity-dependent DNA ligation assays. Nature Biotechnology, 20(5), 473-477. http://doi.org/10.1038/nbt0502-473, which teaches a technique (Proximity Ligation Assay; PLA) in which the coordinated and proximal binding of a target protein by two DNA aptamers promotes ligation of oligonucleotides linked to each aptamer affinity probe. The ligation of two such proximity probes gives rise to an amplifiable DNA sequence that reflects the identity and amount of the target protein. The method 3PLA (Schallmeiner, E. et al., 2007, Sensitive protein detection via triple-binder proximity ligation assays. Nature Methods, 4(2), 135-137. http://doi.org/10.1038/nmeth974) extends the sensitivity and specificity of the proximity ligation method by using three recognition events and allows to detect as little as a hundred target molecules. In 3PLA, sets of three oligonucleotide-modified antibody reagents bind individual target proteins to give rise to a detectable signal by proximity ligation. The 3' and 5' ends of oligonucleotides on two proximity probes are capable of hybridizing to an oligonucleotide present on a third proximity probe forming a complex involving the three probes and the target protein. This allows the two oligonucleotides to be connected via the intermediary fragment by two ligation reactions, templated by the third proximity probe, forming a specific, amplifiable DNA strand that can be detected by qPCR. Proximity Extension Assay (PEA) is a variation of PLA in which 2 oligonucleotide-labeled antibodies bind an individual protein, the oligonucleotides partially anneal at their 3' end and an extension by a polymerase produces an amplifiable DNA sequence which can be detected by qPCR (Lundberg, M. et al., 2011, Homogeneous antibody-based proximity extension assays provide sensitive and specific detection of low-abundant proteins in human blood. Nucleic Acids Research, 39(15). http://doi.org/10.1093/nar/gkr424). While the above disclosed methods were not designed specifically for single-cell protein detection, the Fluidigm C1^{™} single cell auto prep system was employed to automate the preparation of amplifiable targets of a panel of 92 proteins in up to 96 single cells per run using the PEA assay (Egidio C. et al., 2014, A Method for Detecting Protein Expression in Single Cells Using the C1 ™ Single-Cell Auto Prep System (TECH2P.874), J Immunol, 192 (1 Supplement) 135.5). The Fluidigm C1 microfluidic system supports a range of single-cell biology methods for the analysis of transcriptome or genomic DNA sequence by whole exome sequencing and targeted DNA sequencing, however the methods cannot be easily combined to obtain information on genotype and phenotype from the same single cells.

Thus, PLA and PEA assays, in which detection is based on qPCR, are sensitive and highly specific, but are limited in their throughput and can only detect proteins.

US 9,714,937 by NanoString Technologies, Inc. teaches methods for the detection of proteins through the use of a capture antibody conjugated to a moiety, such as biotin, specific for a first region of a target protein and a detection antibody, for a second region of the target protein, with a nanoreporter comprising multiple detachable labels linked to the detection antibody through hybridization to a linker oligonucleotide. The two antibodies form a complex with the target protein which can form a bond with a matrix or a bead with high affinity for the moiety. The target is detected and quantified by counting the number of nanoreporter molecules. Commercially available assays from Nanostring, Inc. based on nCounter^{®} digital molecular barcoding technology, detect proteins using uniquely oligonucleotide-labeled antibodies targeting specific protein epitopes. The unique single-stranded DNA tags are detected using a combination of a biotinylated capture probe and a reporter probe made by a single-stranded DNA molecule annealed to a series of fluorescently labeled RNA segments. The linear order of these labels creates a unique barcode for each target of interest. Complexes are then immobilized to an imaging surface through a non-covalent bond between biotin and immobilized streptavidin molecules and fluorescent barcodes are imaged and counted. The number of counts per protein-specific barcode is a digital measure directly related to the number of molecules present in the sample. Protein detection can be combined to messenger RNAs detection by using capture probe-reporter probe couples designed on specific target RNAs. About 30 protein targets and 770 mRNA targets can be analyzed in a single analysis.

The disadvantages of this method is that it requires large quantities of cells to profile RNAs (equivalent to 2,500 cells) and/or to profile proteins (equivalent to 100,000 cells) and that it is not suitable, as is, to profile single cells. Potentially the method can be used to detect other analytes, such as genomic DNA, however it cannot provide a direct readout of the genomic sequence but only a signal of presence/absence of a known sequence. Being based on hybridization it is also partially tolerant to sequence variants and may not be able to distinguish different sequence variants.

An NGS-based method for integrated analysis of multiple proteins and RNA transcripts in single cells, named cellular indexing of transcriptomes and epitopes by sequencing (CITE-seq), was first described in Stoeckius et al., 2017, Simultaneous epitope and transcriptome measurement in single cells, Nature Methods volume 14, pages 865-868, and US 2018/0251825. The method relies on oligonucleotide-labeled antibodies which are used to integrate cellular protein and transcriptome measurements into a single-cell readout through a 3'-poly adenosine tail present on antibody tags analogous to that present on messenger RNAs. The method is compatible with droplet-based approaches for sample partitioning in single cells and single-cell library preparation, such as that provided by 10X Genomics. In more detail, in the CITE-seq method, cells stained with oligonucleotide-labeled antibodies for cell surface epitopes are partitioned in oil droplets containing lysing enzymes and barcoded beads by microfluidics means. Barcoded antibodies and mRNAs from each single cell/droplet are captured by beads with a unique cell barcode. mRNAs are then retro-transcribed and amplified along with the oligo from barcoded antibodies, generating NGS libraries ready for sequencing. Finally, sequence counts are used for the quantification of barcoded antibodies. Similarly, Peterson et al., 2017, Multiplexed quantification of proteins and transcripts in single cells, Nature biotech., (35) 10:936-939, teaches a method, RNA expression and protein quantification assay (REAP-seq), based on DNA-labeled antibodies and droplet microfluidics, by which proteins can be quantified using 82 barcoded antibodies and the transcription of >20,000 can be profiled in single cells. Both the above mentioned methods exploit the DNA polymerase activity of the reverse transcriptase to simultaneously extend the primed oligo-labeled antibodies with a poly(dT) cell barcode and synthesize complementary DNA from mRNA in the same reaction. On the other hand, other methods, also based on droplet approaches, are available for the analysis of genome-wide copy number profiles or for the analysis of the genome sequence in single cells. For example, the commercial solution Chromium Single cell CNV Solution by 10X Genomics allows copy number profiling of hundreds to thousands of single-cells and Mission Bio's Tapestri^{®} Platform provides single-cell targeted DNA sequencing for sequence and CNV analysis of panels of genes.

The drawbacks of these methods are that:
- both methods for simultaneous transcriptome/proteome profiling described above do not allow the simultaneous analysis of the genome sequence along with proteins or transcripts as genomic DNA does not possess poly-adenosine tails necessary to amplify it.
- Dropseq-based methods for copy number and or targeted sequencing are only suitable for the analysis of genomic DNA but do not provide any information on the phenotype, such as transcription profiles or quantitation of surface markers or other proteins, of the single cells.
- In droplets-based single-cells partitioning approaches, single cells and all their informational content are essentially "destroyed" in the process, and it is not possible to recover the single cells, after the procedure is completed, to perform further analyses.

US 2019/360044 discloses protein quantification in single cells by preparing hydrogel droplets, and delivering oligonucleotide linked antibodies to the hydrogel droplets. Each oligonucleotide linked antibody may comprise a UPS, a reverse priming site, a UMI, and an antibody identifying barcode.

WO 2018/144813 discloses compositions and methods comprise one or more constructs, each construct comprising a ligand attached or conjugated to a polymer construct, e.g., an oligonucleotide sequence, by a linker, each ligand binding specifically to a single target located in or on the surface of a cell. The polymer construct comprises a) an Amplification Handle; b) a Barcode that specifically identifies a single ligand; c) an optional Unique Molecular Identifier that is positioned adjacent to the Barcode on its 5' or 3' end; and d) an Anchor for hybridizing to a complementary sequence, e.g., for generation of a doublestranded oligonucleotide. These compositions are used in high throughput methods for detecting one or more targets or epitopes in a biological sample and for characterizing a cell by simultaneous detection of one or more epitopes located in or on the cell and its transcriptome.

### Summary of the Invention

It is an object of the present invention to provide a method for whole genome amplification and analysis of multiple target molecules in a biological sample that simultaneously allows analysis of genome-wide copy-number profiles/genome sequence and analysis of protein expression on the same single cells, overcoming in particular one or more of the following drawbacks of the state of the art:
- impossibility to detect and quantify proteins and analyze genome in the same sample down to single-cell resolution,
- impossibility to reanalyse a single cell for additional targeted genomic information.

This object is achieved by the method as defined in claim 1.

A further object of the present invention is to provide a kit as defined in claim 17.

### Brief Description of the Drawings

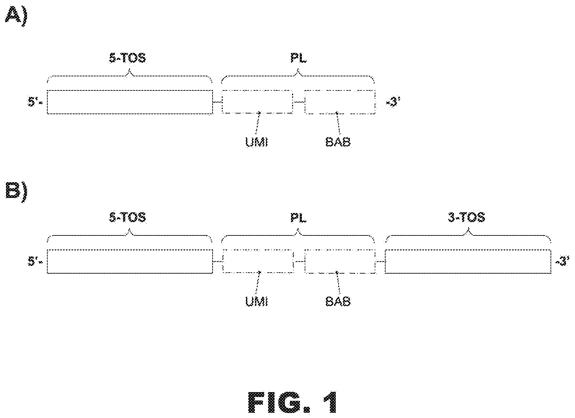

Figure 1 shows the general structure of two possible embodiments of a tagged oligonucleotide without (Figure 1A) or with (Figure 1B) a 3' tagged oligo sequence according to the invention. PL = payload sequence; 5-TOS = first tagged oligonucleotide amplification sequence; 3-TOS = second tagged oligonucleotide amplification sequence; UMI = unique molecular identifier sequence; BAB = binding agent barcode sequence.

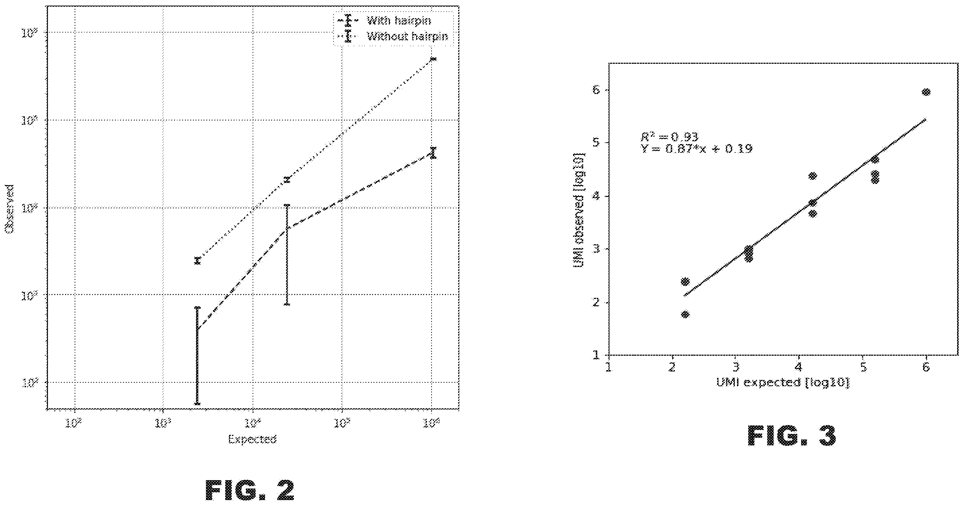

Figure 2 shows a graph that represents tagged oligonucleotide libraries from two distinct tagged oligos, one prone to intramolecular hairpin formation between 5-TOS and 3-TOS at different temperatures ("with hairpin": Tm = 67°C; "without hairpin": Tm = 45°C). On the x-axis the expected UMI count, on the y-axis the UMIs counted after sequencing.
Figure 3 shows a graph that represents the amplification of oligo mixtures in different quantities with 27 PCR cycles. Each dilution was performed from three independent dilution replicates. On the x-axis the number of distinct UMIs expected to be observed, on the y-axis the experimentally observed UMIs.

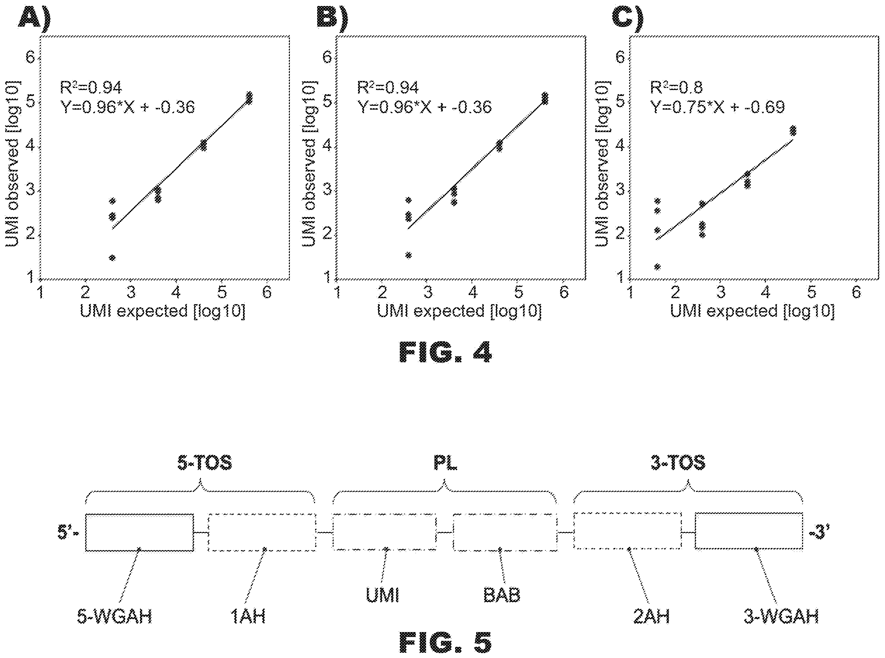

Figure 4 shows three graphs of amplification of four oligo mixtures with different BAB in different quantities. Each dilution has four data points, one for each oligo. Figure 4A: performed 23 PCR cycles. Figure 4B: performed 27 PCR cycles. Figure 4C: performed 35 PCR cycles. On the x-axis the number of distinct UMIs expected to be observed, on the y-axis the experimentally observed UMIs.
Figure 5 shows the structure of an embodiment according to the invention of the tagged oligo with one primer amplification. Additional captions: 5-WGAH = 5' WGA handle sequence; 3-WGAH = 3' WGA handle sequence; 1AH = first amplification handle sequence; 2AH = second amplification handle sequence.

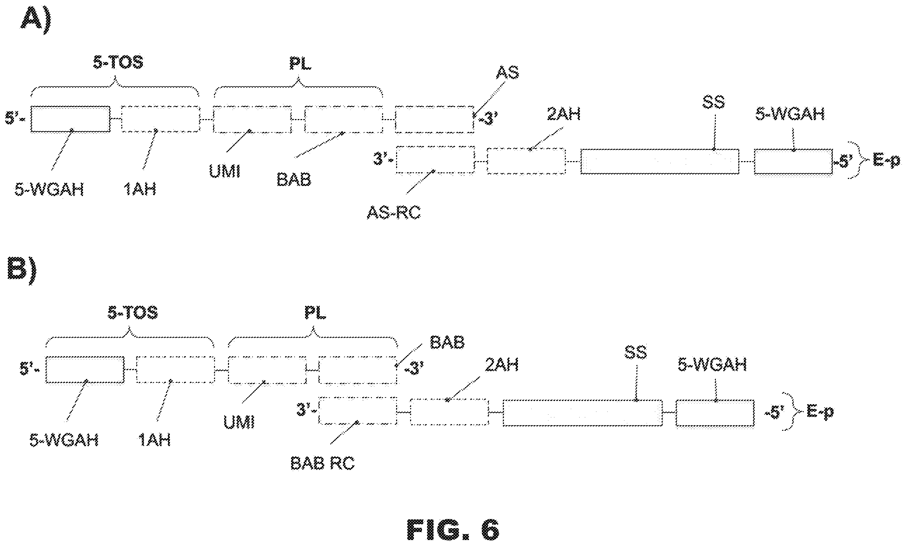

Figure 6 shows the structure of another embodiment according to the invention of the tagged oligo with at least a second primer amplification. Figure 6A: structure of tagged oligo and relative extension oligonucleotide with annealing site corresponding to the BAB. Figure 6B: structure of tagged oligo and relative extension oligonucleotide with annealing site not corresponding to the BAB. Additional captions: E-p = 5' extension oligonucleotide sequence; SS = spacer sequence; AS = annealing sequence; AS-RC = annealing sequence reverse complement.

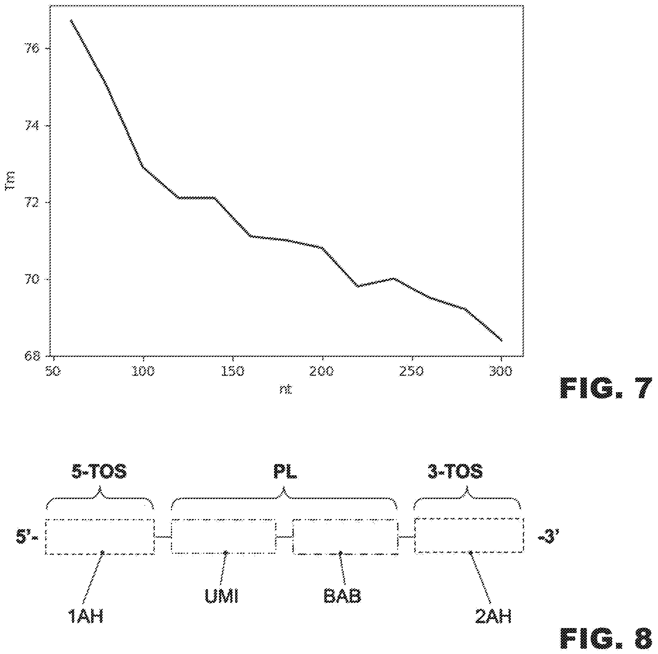

Figure 7 shows a graph of the *in silico* prediction of the melting temperature ([Na⁺]=150mM; [Mg⁺⁺]=4mM) of a hairpin induced by 15 nt long complementary sequences located at the end of a ssDNA molecule as a function of molecule length.
Figure 8 shows the structure of another embodiment according to the invention of the tagged oligo with an at least a three primer amplification.

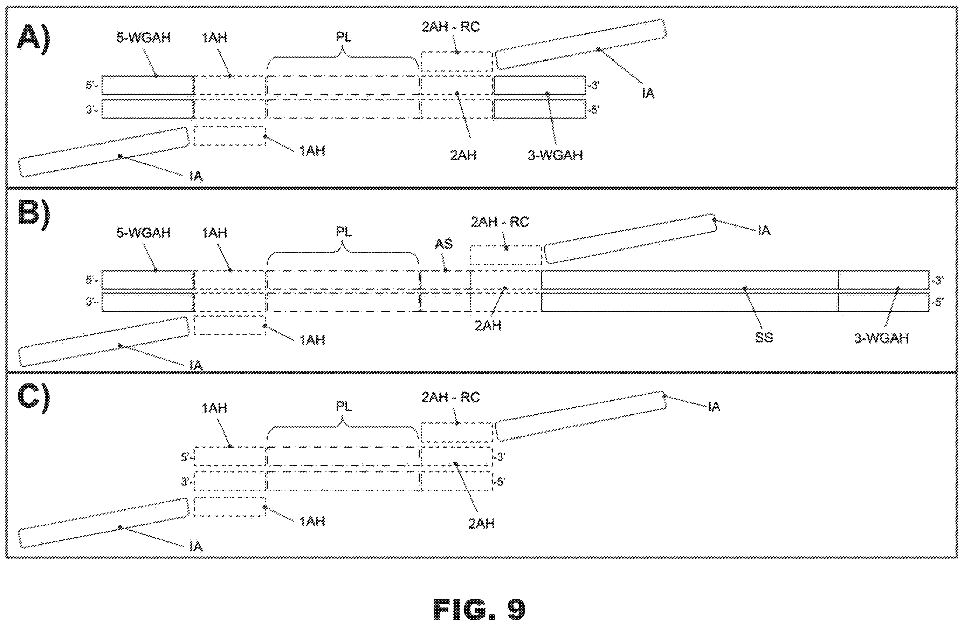

Figure 9 shows the general scheme for library generation. Figure 9A: generation of library from tagged oligo according to the embodiment of Figure 5. Figure 9B: generation of library from tagged oligo according to the embodiment of Figure 6A. Figure 9C: generation of library from tagged oligo according to the embodiment of Figure 8. Additional caption: 2AH-RC = second amplification handle sequence reverse complement.

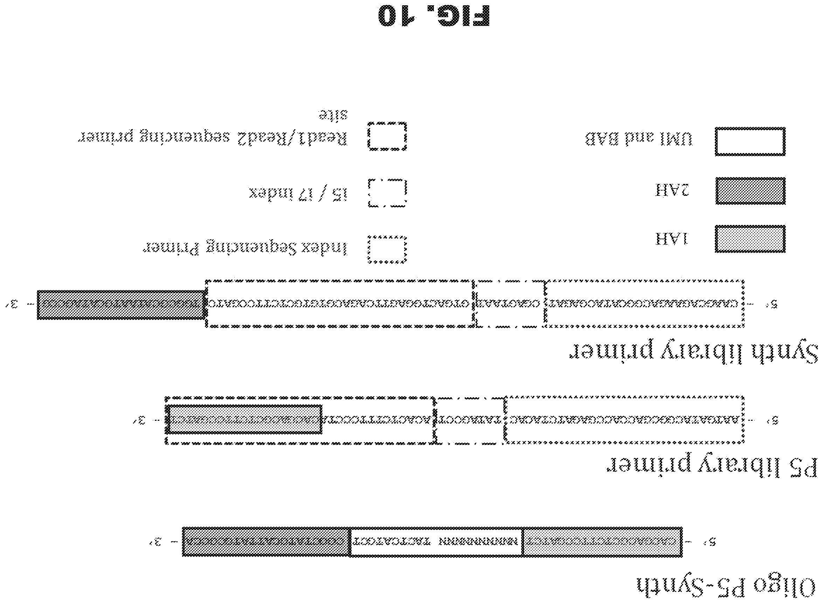

Figure 10 shows the design of P5-Synth oligo and corresponding library primers disclosed in Example 1.

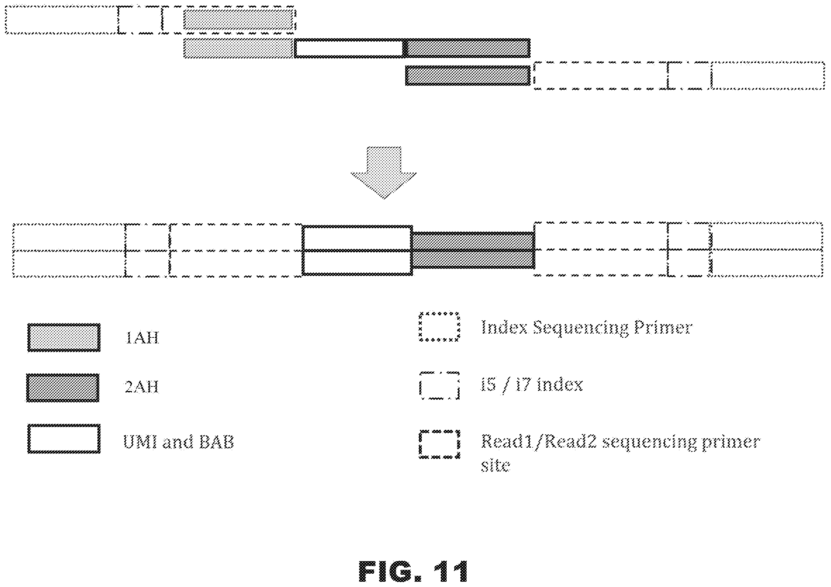

Figure 11 shows the scheme of NGS library generation of Oligo P5-Synth using library primers according to Example 1.

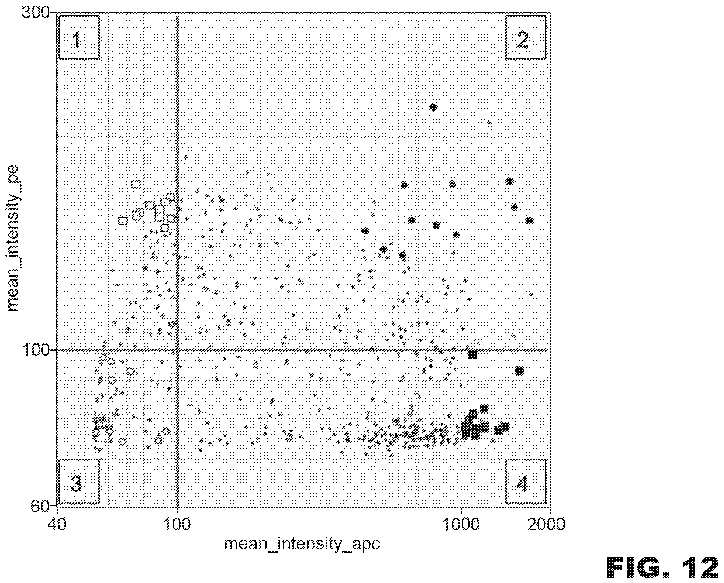

Figure 12 shows a scatterplot of PBMC and SK-BR-3 cells stained with Ab-oligo and secondary fluorescent antibodies. On the x-axis the fluorescence levels in the APC channel, which is proportional to the quantity of Ab-oligo tag1, tag2 and tag4. On the y-axis the fluorescence levels in the PE channel, which is proportional to the quantity of Ab-oligo tag3.

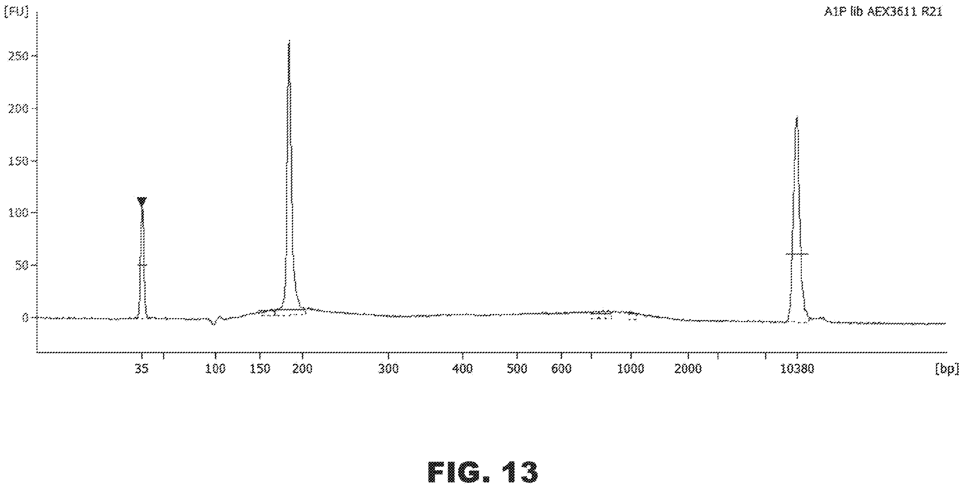

Figure 13 shows an electropherogram from a library generated from P5-synth tagged oligo from single cells according to Example 1.

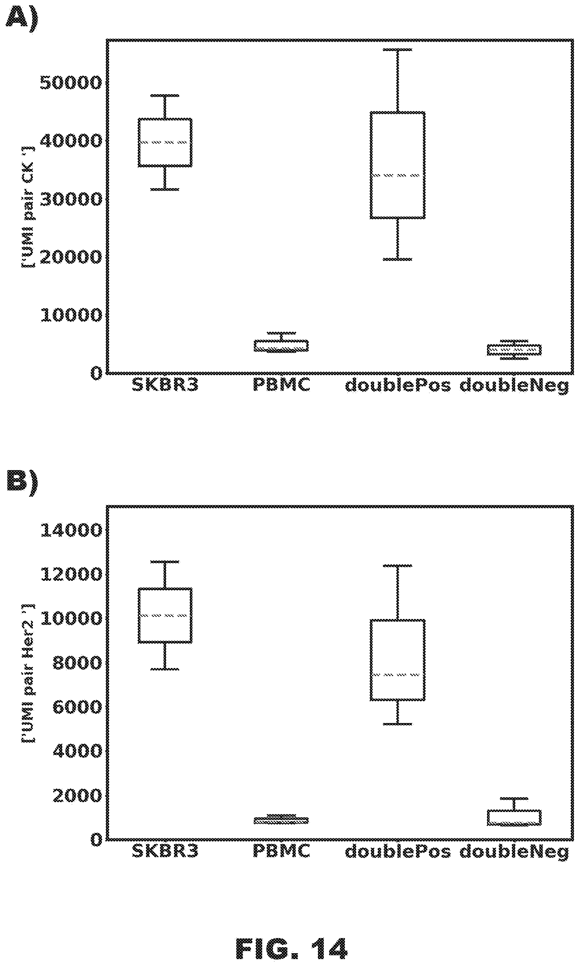

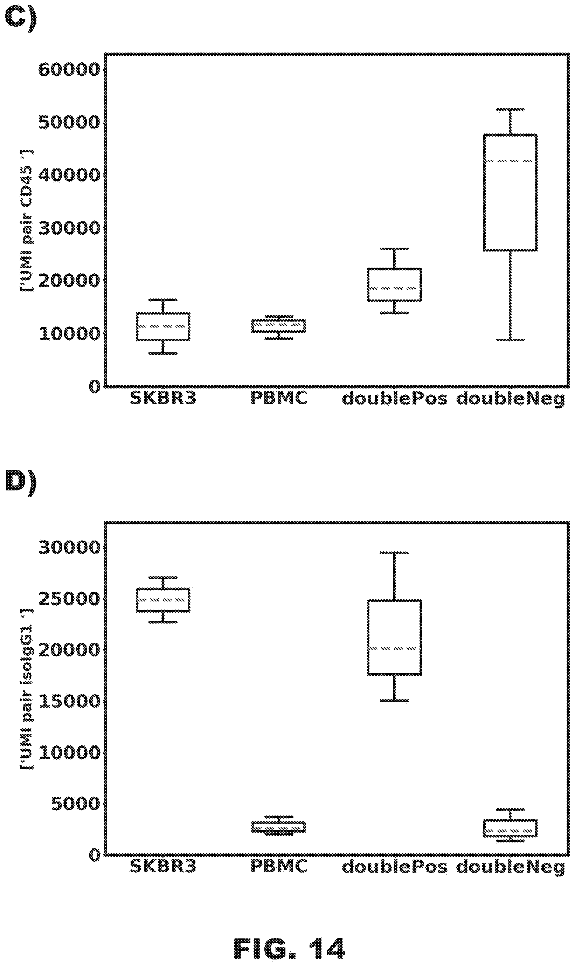

Figure 14 shows the protein quantification results from single cells processed according to the embodiment of Figure 8 with tagged oligo amplification after WGA. UMI counts of cytokeratin (Figure 8A), Her2 (Figure 8B), CD45 (Figure 8C) and IgG1 isotype control (Figure 8D) quantification respectively. On the y-axis the number of UMI, on the x-axis the cell types isolated according to Figure 9.

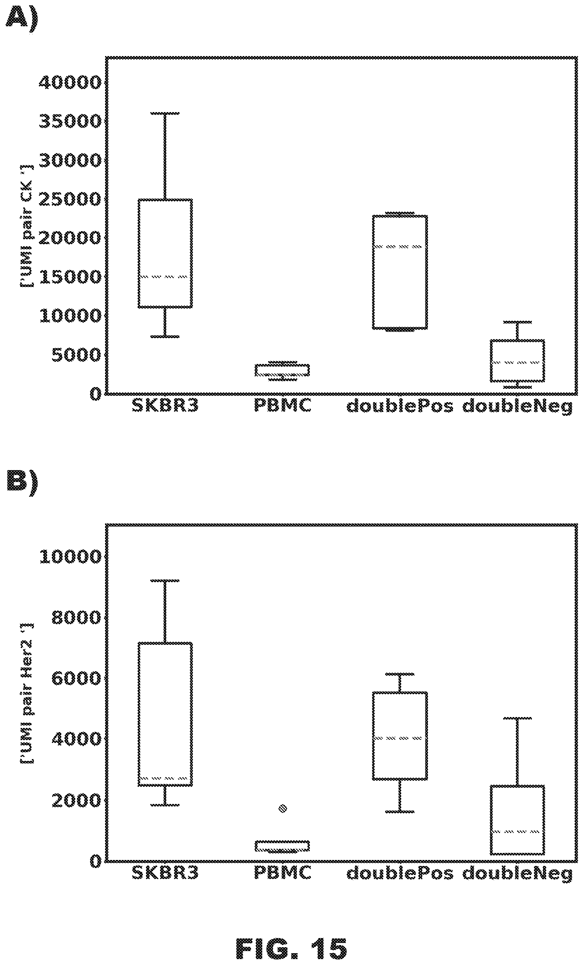

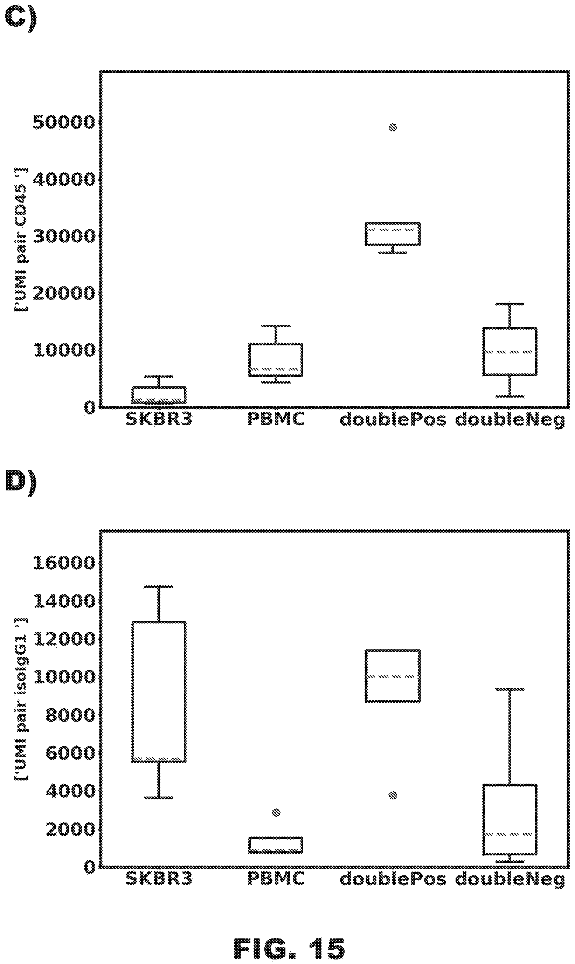

Figure 15 shows the protein quantification results from single cells processed according to the embodiment of Figure 8 with tagged oligo amplification during WGA. UMI counts of cytokeratin (Figure 8A), Her2 (Figure 8B), CD45 (Figure 8C) and IgG1 isotype control (Figure 8D) quantification respectively. On the y-axis the number of UMI, on the x-axis the cell types isolated according to Figure 9.

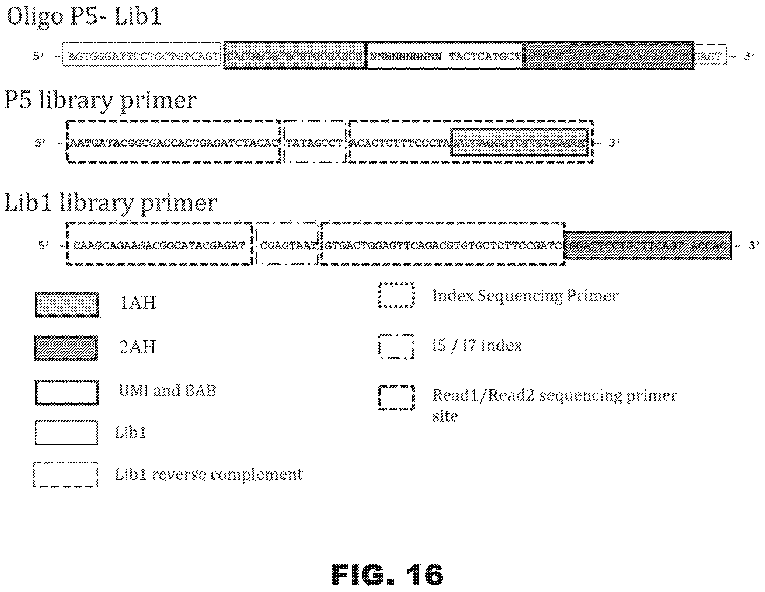

Figure 16 shows the design of P5-Lib1 oligo and corresponding library primers disclosed in Example 3.

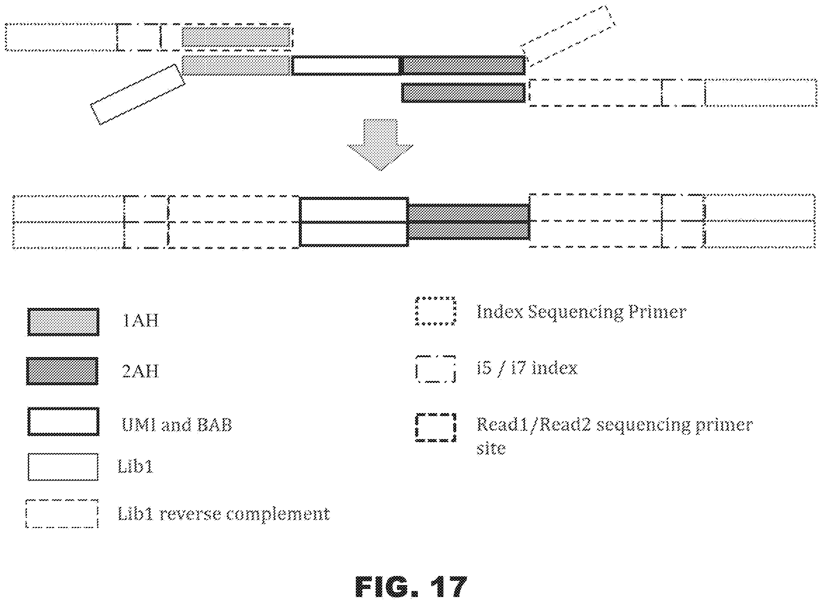

Figure 17 shows the scheme of NGS library generation of P5-Lib1 oligo using library primers.

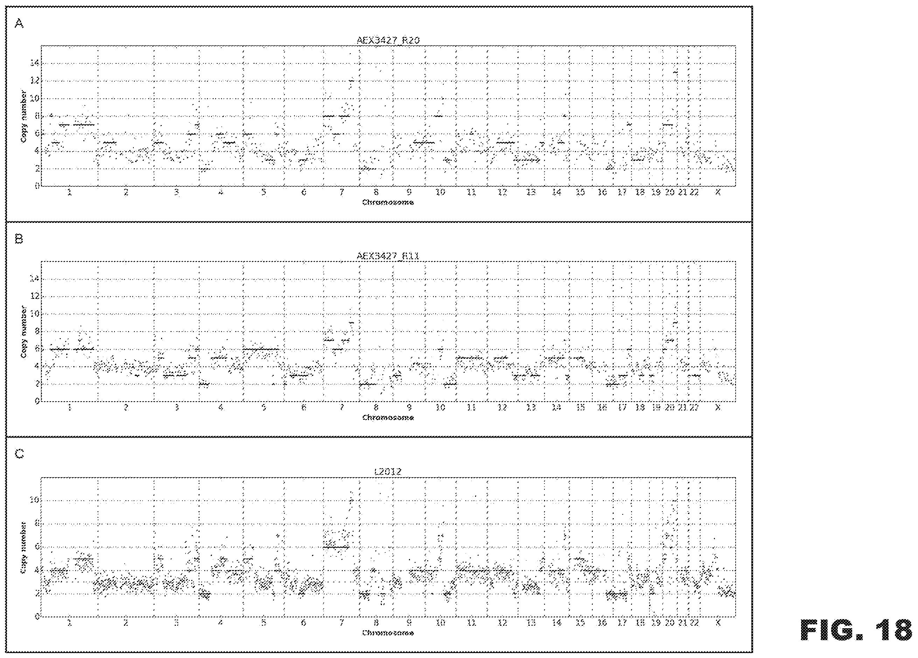

Figure 18 shows examples of LowPass profiles for CNA analysis obtained from a single cell. Figure 18A: single cell CNA profiles that was spiked with P5-Lib1 oligo and processed according to the embodiment of Figure 5. Figure 18B: single cell CNA profiles that was spiked with P5-Synth oligo and processed according to the embodiment of Figure 8. Figure 18C: single cell CNA profiles without tagged oligo spiking. All profiles correspond to SK-BR-3 cell with typical gain and losses. Small variations are due to single cell genomic heterogeneity.

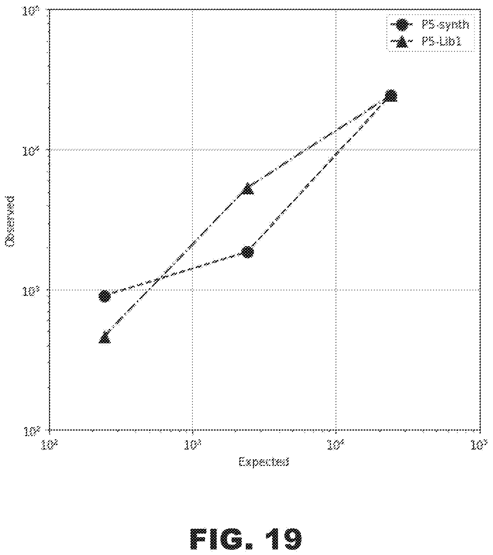

Figure 19 shows a graph representing tagged oligo libraries obtained from single cells spiking with P5-Synth and P5-Lib1. On the x-axis the expected UMI count, in the y-axis the UMIs counted after sequencing.

### Definitions

Unless defined otherwise, all technical and scientific terms used herein have the same meaning as commonly understood by one of ordinary skill in the art to which this invention pertains. Although many methods and materials similar or equivalent to those described herein may be used in the practice or testing of the present invention, preferred methods and materials are described below. Unless mentioned otherwise, the techniques described herein for use with the invention are standard methodologies well known to persons of ordinary skill in the art.

By "ab-oligo mix" there is intended a solution containing all ab-oligos that target a cell's internal (namely "internal ab-oligo mix") and/or external (namely "external ab-oligo mix") epitopes and/or isotypic control ab-oligos.

By "antibody-oligonucleotide conjugate" or "ab-oligo conjugate" or "ab-oligo" there are intended synthetic molecules derived by the chemical conjugation of an antibody molecule with ssDNA oligonucleotide molecules. Chemical conjugation is usually performed using specific chemical reactions that enable the linking of the two molecules in a covalent manner. The antibody:oligonucleotide stoichiometry can be controlled in order to have a specified ratio. During WGA initial steps, the antibody moiety is usually digested and only the oligonucleotide part remains. For the sake of simplicity, in the description these molecules will still be referred to as ab-oligo molecules or ab-oligo amplicons.

By the acronym "APC" there is intended the fluorophore allophycocyanin.

By "binding agent barcode sequence (BAB)" there is intended a unique DNA oligonucleotide sequence that identifies the binding agent.

By "balanced PCR amplification" there is intended the feature of a PCR to perform an amplification of multiple targets whereby, in each PCR cycle, substantially every target molecule is amplified.

By "binding agent" there is intended a molecule (such as, by way of a non-limiting example, antibodies, affibodies, ligands, aptamers, synthetic binding proteins, small molecules) able to specifically bind designated target molecules, for example proteins or glycosylated proteins or phosphorylated proteins.

By "CITE-Seq" or "Cellular Indexing of Transcriptomes and Epitopes by Sequencing" there is intended the method developed by Stoeckius et al. for simultaneous protein quantification and mRNA sequencing in single-cells.

By "CyTOF" or "cytometry by time of flight" there is intended an equipment carrying out mass-cytometry technique, enabling the quantification of proteins in single cells using mass spectrometry in combination with cytometry. Cells are stained with binding agents conjugated with heavy metal isotopes.

By the term "conjugate" there is intended a molecule obtained from the covalent conjugation of a binding agent and a tagged oligonucleotide.

By "copy number alteration (CNA)" there is intended a somatic change in copy-numbers of a genomic region, defined in general with respect to the same individual genome.

By "DNA library purification" there is intended a process whereby the DNA library material is separated from unwanted reaction components such as enzymes, dNTPs, salts and/or other molecules which are not part of the desired DNA library. Examples of DNA library purification processes are purification with Agencourt AMPure, or Merck Millipore Amicon spin-columns or solid-phase reversible immobilization (SPRI)-beads such as from Beckman Coulter.

By "DNA library quantification" there is intended a process whereby the DNA library material is quantified. Example of DNA library quantification processes are quantification using QuBit technology, electrophoretic assays (Agilent Bioanalyzer 2100, Perkin Elmer LabChip technologies) or RT-PCR PicoGreen system (Kapa Biosystems).

By "dynamic range" there is intended the ratio between the largest and smallest values that a certain quantity can assume.

By "library primers" there is intended ssDNA molecules that act as primers in order to generate massively-parallel sequenceable libraries from tagged oligonucleotides.

By "low-pass whole genome sequencing" or "lowpass sequencing" there is intended a whole genome sequencing at mean sequencing depth lower than 1.

By "massive-parallel sequencing" or "next generation sequencing (NGS)" there is intended a method of sequencing DNA comprising the creation of a library of DNA molecules spatially and/or time separated, clonally sequenced (with or without prior clonal amplification). Examples include Illumina platform (Illumina Inc), IonTorrent platform (ThermoFisher Scientific Inc), Pacific Biosciences platform, MinIon (Oxford Nanopore Technologies Ltd).

By "Multiple Annealing and Looping Based Amplification Cycles (MALBAC)" there is intended a quasilinear whole genome amplification method (Zong et al., 2012, Genome-wide detection of single-nucleotide and copy-number variations of a single human cell, Science. Dec 21;338(6114):1622-6. doi: 10.1126/science.1229164.). MALBAC primers have an 8 nucleotide 3' random sequence to hybridize to the template, and a 27 nucleotides 5' common sequence (GTG AGT GAT GGT TGA GGT AGT GTG GAG). After first extension, semiamplicons are used as templates for another extension yielding a full amplicon which has complementary 5' and 3' ends. Following few cycles of quasi-linear amplification, full amplicon can be exponentially amplified with subsequent PCR cycles.

By the term "oligonucleotide" or "oligo" there is intended an oligomeric molecule comprising a sequence of nucleotides such as, by way of non-limiting example, deoxyribonucleic acid (DNA), ribonucleic acid (RNA), locked nucleic acid (LNA), peptide nucleic acid (PNA).

By "tagged oligonucleotide" or "tagged oligo" there is intended an oligonucleotide molecule (e.g. ssDNA molecule) that is directly conjugated to a binding agent (e.g. a primary antibody). The tagged oligo is used for indirect quantification of the target-molecule (e.g. a protein) which is a ligand for the binding agent.

By the acronym "PE" there is intended the fluorophore phycoerythrin.

By "PFA 2%" there is intended a solution at 2% w/v of paraformaldehyde in phosphate buffer saline.

By "primary WGA DNA library (pWGAlib)" there is intended a DNA library obtained from a WGA reaction.

By the term "re-amplification" or "re-amp" there is intended a PCR reaction where all or a substantial portion of the primary WGA DNA library is further amplified.

By "residues" there are intended the amino acid residues present in polypeptide chains in proteins.

By "sequencing barcode" there is intended a polynucleotide sequence which, when sequenced within one sequencer read, allows to assign that read to a specific sample associated with that barcode.

By "UMI" or "Unique Molecular Identifier sequence" there is intended degenerate or partially-degenerate (i.e. random or semi-random) oligonucleotide sequences which are virtually unique for each ssDNA or dsDNA molecule.

By "universal WGA-primer" or "WGA PCR Primer" there is intended the additional oligonucleotide ligated to each fragment generated by the action of the restriction enzyme. Universal WGA-primer are used in DRS-WGA such as Ampli1^{™} WGA

### Detailed Description of the Invention

The method for whole genome amplification and analysis of multiple target molecules in a biological sample including genomic DNA and target molecules according to the present invention comprises the following steps.

In step a), the biological sample is provided. The biological sample is preferably a single cell, but can also be a sample comprising several cells.

In step b), the biological sample is contacted with at least one binding agent, which is directed to at least one of the target molecules, conjugated with a tagged oligonucleotide, so that - when at least one target molecule is present in the biological sample - the at least one binding agent binds to the at least one target molecule.

The binding agent is preferably selected from the group consisting of an antibody or fragment thereof, an aptamer, a small molecule, a peptide, and a protein. The target molecule is preferably selected from the group consisting of a protein, a peptide, a glycoprotein, a carbohydrate, a lipid, and a combination thereof. More preferably, the binding agent is an antibody. Binding agents bind target molecules with specific stoichiometry such as monoclonal antibodies or enzyme substrates, or with unspecified stoichiometry such as polyclonal antibodies or small molecules. The former enable a better quantification of the target with respect to the latter. Binding agents are chemically conjugated to tagged oligos via covalent bond interactions or non-covalent interactions. In the former case, both oligos and binding agents possess reactive moieties which enable reciprocal binding. Binding agent:oligo stoichiometry can be controlled during conjugation procedures.

A non limiting list of examples of binding-agent / target molecules is reported in the following Table 1.

**Table 1**

| **Binding agent(s)** | **Target molecule(s)** |
|---|---|
| Antibody or fragment of antibody such as nanobody or Fab | Antigen, post-translationally modified proteins, such as phospho-proteins (e.g. pAKT), or acetylated proteins (e.g. Histones) |
| Aptamer | Antigen |
| Small molecule a drug | Cell surface receptor, channel |
| Peptide | protein, enzymes |
| Protein | Protein, DNA, RNA |

Oligonucleotides used as tagged oligonucleotides are preferably ssDNA or dsDNA molecules with a chemical modification on the 5' or 3' end. This modification is used for the covalent conjugation with the relative binding agent.

Conjugates formed by the tagged oligos conjugated with the binding agents may target both extra-cellular and intracellular epitopes. "External" and "internal" conjugates can be applied to the biological sample as two separate mixes containing the conjugates at their final staining concentration. Firstly, an external mix is applied to label external epitopes. Secondly, cells are permeabilized using detergents or similar means and an internal mix is applied to the sample to label internal epitopes. Alternatively, external and internal conjugates can be mixed together as well to perform a one-step staining. The final staining concentration varies for each binding agent and must be determined experimentally.

The tagged oligo sequence is preferably shorter than 300 bases, more preferably shorter than 120 bases to facilitate conjugation with the binding agent and to reduce cost. In a preferred embodiment, the tagged oligo sequence is between 60-80 nucleotides.

With reference to Figure 1A, the tagged oligonucleotide comprises:
i) a payload sequence of nucleic acid (PL) comprising a binding-agent barcode sequence (BAB) and a unique molecular identifier sequence (UMI), and
ii) at least one first tagged oligonucleotide amplification sequence of nucleic acid (5-TOS).

The payload sequence contains the necessary information for target counting.

The unique molecular identifier sequence (UMI) is preferably a degenerate or semi-degenerate sequence in the range from 10 to 30 nucleotides. Preferably the UMI has a length of at least 10 bases, corresponding to a theoretical 4^10=1,048,576 different combinations, which is enough for most target molecules. For highly abundant target molecules, a longer UMI may be used to increase the possible combinations, such as 12 bases. Using semi-degenerate bases, the possible combination decrease and the UMI length is preferably increased, for example up to 20 or 30 bases. Semi-degenerate UMIs may be advantageously used for introducing reference points which can be used in the read-out to realign the sequence preventing an overestimation of the distinct UMIs present. The UMI sequence may be located at either the 5' or the 3' of the BAB. The UMI sequence is preferentially located just after the Read 1 sequencing primer annealing site to increase the complexity of the first sequenced bases. This is advantageous for Illumina sequencing platforms as initial sequencing steps require high complexity for cluster discrimination. BAB is a fixed sequence for each conjugate molecule. BAB are designed in order to avoid features which may interfere with primary PCR amplification and sequencing steps, such as homopolymers, hairpins, and/or heteroduplex formation [Frank, D. N., 2009, BARCRAWL and BARTAB: software tools for the design and implementation of barcoded primers for highly multiplexed DNA sequencing. BMC Bioinformatics, 10, 362. http://doi.org/10.1186/1471-2105-10-362] and are selected from the pool of all possible BAB sequences of a defined length in order to maximize their relative Hamming distance, thus minimizing the possibility that any PCR or sequencing error might lead to an incorrect assignment of the sequenced read. BAB length must be selected based on the number of target molecules to be detected. Preferably the BAB has a length of at least 10 nucleotides, corresponding to theoretical 4^10=1,048,576 different combinations which are reduced to about 2000 possible BAB sequences after applying filters on GC content (for example: [30%..70%]), absence of homopolymers, absence of hairpins and minimum hamming distance (preferably ≥3 nt).

The first tagged oligonucleotide amplification sequence (5-TOS) is located at the 5' of the tagged oligo. This sequence is required for tagged oligo amplification and subsequent library generation. Tagged oligo amplification is necessary to avoid any bias due to loss of molecules while processing samples which may hamper proper UMI counting.

With reference to Figure 1B, the target oligonucleotide preferably further comprises at least one second tagged oligonucleotide amplification sequence (3-TOS). This sequence is located at the 3' of the tagged oligo. This sequence is required for tagged oligo amplification and subsequent library generation. Tagged oligo amplification is necessary to avoid any bias due to loss of molecules while processing samples which may hamper proper UMI counting.

In a preferred embodiment 5-TOS and 3-TOS sequences are designed to avoid the formation of hairpins and other intramolecular stable secondary structures within the amplification temperature range, as they may hamper tagged oligo amplification. Figure 2 shows a graph that represents tagged oligonucleotide libraries from two distinct tagged oligos, one prone to intramolecular hairpin formation between 5-TOS and 3-TOS at different temperatures ("with hairpin": Tm = 67°C, SEQ ID NO:50, ΔG = -11.15 kcal/mol; "without hairpin": Tm = 45°C, SEQ ID NO:51, ΔG = -1.52 kcal/mol). On the x-axis the expected UMI count, on the y-axis the UMIs counted after sequencing.

Tagged oligos and their amplification primers are optimized to achieve high sensitivity, extensive dynamic range, balanced PCR amplification and reproducibility.

In preferred embodiments of the present invention the UMI sequence length was selected (n = 10) to quantify targets in the range of 0 to ~10⁶ molecules.

Dynamic range was characterized by amplification of tagged oligos with a concentration spanning four orders of magnitude (from 10² to 10⁶ molecules). Figure 3 shows a graph representing the amplification of oligo mixtures in different quantities with 27 PCR cycles. Each dilution was performed from three independent dilution replicates. On the x-axis the number of distinct UMIs expected to be observed, on the y-axis the experimentally observed UMIs. A highly linear correlation in the range of 10² to 10⁶ number of molecules between the observed UMIs and expected UMIs prior amplification can be observed.

Balanced PCR amplification was characterized by performing different cycles of amplification on the same starting sample. As shown in Figure 4A and 4B, amplification of the same pool of tagged oligos with a different number of total PCR cycles (respectively 23 and 27 PCR cycles) did not result in a difference in UMIs observed, indicating that the number of PCR cycles does not affect UMI counting.

Sensitivity was characterized by amplification of a pool of tagged oligos in different quantities (down to 40 molecules) . As shown in Figure 4C, it is possible to quantify down to 10² tagged oligo molecules. It should be noted that serial diluted solution experiments are prone to sampling biases due to highly heterogeneous distribution of molecules within the volume and this is especially relevant with very diluted solutions. Thus the limit of quantification observed might be an underestimate related to the experimental setup rather than the limitations of the assay.

In step c) of the method according to the invention, a separating step is carried out to selectively remove unbound binding agent, thus obtaining a labeled biological sample. The separating step is typically carried out by washing in a suitable buffer solution and collecting the labelled biological sample by centrifugation.

In step d), a whole genome amplification of said genomic DNA and an amplification of the tagged oligonucleotide conjugated with the at least one binding agent are carried out simultaneously on the labeled biological sample. The whole genome amplification of the genomic DNA is carried out by either deterministic restriction-site whole genome amplification (DRS-WGA), or by Multiple Annealing and Looping Based Amplification Cycles whole genome amplification (MALBAC).

In step e) a massively parallel sequencing library is prepared from the amplified tagged oligonucleotide.

In step f), the massively parallel sequencing library is sequenced.

In step g), the sequences of the binding-agent barcode sequence (BAB) and unique molecular identifier sequence (UMI) are retrieved from each sequencing read.

In step h), the number of distinct unique molecular identifier sequences (UMI) is counted for each binding agent.

Steps e), f), g) and h) will be disclosed in greater detail with reference to specific embodiments later on in the description.

The above-disclosed method preferably further comprises a step of isolating a single cell from the biological sample. Isolation may be carried out by sorting cells, in particular e.g. using a cell sorter such as DEPArray^{®} NxT (Menarini Silicon Biosystems S.p.A.), or - as an alternative - by partitioning cells into droplets. The step of isolating is preferably performed after step c) and before step d).

The above-disclosed method preferably comprises a step of purifying the massively parallel sequencing library before step f).

In more specific terms but with no intent to limit the scope of the present description, the above-disclosed method, also designated as Ampli1 Protein (A1-P), allows the quantification of proteins and whole genome genetic characterization of single cells. Single or multiple proteins quantification in single-cells is achieved using a panel of binding agents (in particular, antibodies (Ab)) conjugated with tagged oligonucleotides. These oligonucleotides are designed to unambiguously identify the conjugated antibody by means of a DNA barcode sequence and to quantify the abundance of the epitope of interest by means of a random or partially degenerate sequence, namely Unique Molecular Identifiers (UMI), which is used for epitope quantification. Biological samples are labeled with one or more Ab-oligo conjugates, each with a unique DNA barcode sequence. Subsequently, single cells or pools of cells can be isolated by different means (i.e DEPArray^{™} NxT system) and their genomic content can be amplified by whole genome amplification (i.e Ampli1^{™} Whole-Genome-Amplification kit). During the latter step o right after it, tagged oligonucleotides are pre-amplified to avoid any downsampling during NGS library preparation procedure. Specific primers, namely "library primers" are used to generate NGS (Illumina) libraries ready to be sequenced. Tagged Oligonucleotides are designed to be compatible with the Ampli1^{™} WGA (A1-WGA) workflow, enabling single-cell genetic analyses (for example Ampli1^{™} LowPass) in parallel with protein quantification using A1-P.

In the following, three specific embodiments of the present invention are disclosed, which respectively employ a different number of primers for tagged oligo amplification and whole genome amplification.

In a first preferred embodiment and with reference to Figure 5, the tagged oligonucleotide comprises from 5' to 3' at least:
a) the first tagged oligonucleotide amplification sequence of nucleic acid (5-TOS), comprising in turn a 5' whole genome amplification handle sequence (5-WGAH) and a first amplification handle sequence (1AH);
b) the payload sequence (PL);
c) the second tagged oligonucleotide amplification sequence of nucleic acid (3-TOS), comprising in turn a second amplification handle sequence of nucleic acid (2AH) and a 3' whole genome amplification handle sequence (3-WGAH).

The 3-WGAH is the reverse complementary sequence of 5-WGAH enabling simultaneous amplification of gDNA and tagged oligonucleotides during whole genome amplification. The 1AH and the 2AH are located at the 5' and 3' ends of the payload sequence, respectively, and are used for subsequent library generation. The 1AH and 2AH are preferably designed to avoid stable intramolecular secondary structures, such as hairpins, which may inhibit tagged oligo amplification. Between each aforementioned sequence, fixed sequences may be present.

The whole genome amplification and the amplification of the tagged oligo are preferably carried out using a single primer.

In a second preferred embodiment and with reference to Figures 6A and 6B, the tagged oligonucleotide comprises from 5' to 3' at least:
a) the first tagged oligonucleotide amplification sequence of nucleic acid (5-TOS), comprising in turn a 5' whole genome amplification handle sequence (5-WGAH) and a first amplification handle sequence (1AH);
b) the payload sequence (PL);
c) optionally, an annealing sequence (AS).

At least one primer is used for whole genome amplification and amplification of the tagged oligonucleotide and at least one oligonucleotide (E-p) is used for the extension of the tagged oligonucleotide, said at least one oligonucleotide (E-p) comprising from 5' to 3' at least:
d) the 5' whole genome amplification handle sequence (5-WGAH);
e) a spacer sequence (SS);
f) a second amplification handle sequence (2AH); and
g) a sequence reverse complementary to the annealing sequence (AS-RC) or a sequence reverse complementary to the binding-agent barcode sequence (BAB-RC).

In other words, amplification of the tagged oligo occurs by annealing of E-p to AS located at the 3' of the tagged oligo (Figure 6A), by means of an annealing sequence reverse complement (AS-RC), located at the 3' end of E-p, thus causing within the reaction a 3' extension of both the tagged oligo and the E-p, generating in turn WGA-primer amplifiable molecules. Alternatively, AS can coincide with the BAB sequence and E-p anneals to the BAB sequence via BAB reverse complement sequence (BAB-RC) as indicated in Figure 6B. The first option (annealing to AS) has the advantage that a single E-p can be used with any BAB, thus reducing manufacturing costs and protocol complexity. The second option (annealing to BAB) may be advantageously used to normalize the signal deriving from targets having large differences in abundance. This may be achieved, as non-limiting example, by using limiting amounts of primer for potentially highly abundant targets, or using different BAB annealing temperatures, to reduce the amplification of highly abundant tagged oligos. The annealing temperature may be tuned by the BAB length and/or composition.

After the extension of the tagged oligo and E-p, the WGA primer will perform the amplification of tagged oligos, within the resulting larger molecule. The spacer sequence (SS) increases the length of the amplicons generated by the tagged oligos. The increased fragment length destabilizes intramolecular secondary structures, such as hairpins induced by the fragment's complementary ends, thus decreasing their melting temperature (Figure 7), favoring the tagged oligo amplification along with the other WGA fragments (M. Zuker. Mfold web server for nucleic acid folding and hybridization prediction. Nucleic Acids Res. 31 (13), 3406-15, (2003)). The extension oligonucleotide (E-p) sequence length is preferably within the range 60-300 bases. More preferably the extension oligonucleotide (E-p) sequence length is within the range 120-200 bases.

In a third preferred embodiment and with reference to Figure 8, the tagged oligonucleotide comprises from 5' to 3' at least:
a) the first tagged oligonucleotide amplification sequence of nucleic acid (5-TOS) corresponding to a first amplification handle sequence (1AH);
b) the payload sequence (PL);
c) the second tagged oligonucleotide amplification sequence of nucleic acid (3-TOS) corresponding to a second amplification handle sequence (2AH).

At least one first primer is used for whole genome amplification and at least one second and one third primer are used for the amplification of the tagged oligonucleotides, the at least one second primer having a sequence identical to the first amplification handle sequence (1AH) and the at least one third primer having a sequence reverse complementary to the second amplification handle sequence (2AH-RC).

Tagged oligo amplification primers are designed to have a melting temperature that is compatible with at least the first 10-15 cycles of the WGA PCR thermal profile.

Preferably, the at least one second primer and at least third primer are added in step d).

Step e) of preparing a massively parallel sequencing library from the amplified tagged oligonucleotide is preferably performed by a PCR reaction using at least one first library primer, comprising a 3' sequence corresponding to the first amplification handle sequence (1AH) and at least one second library primer comprising a 3' sequence corresponding to the sequence reverse complementary to the second amplification handle sequence (2AH-RC).

Library primers are therefore advantageously used to generate NGS libraries for binding agent quantification analysis with a single PCR step. The specific examples of library primers reported in the present description are used to generate libraries compatible with the Illumina sequencing platform, without this intending to limit the scope of invention of the present invention.

In a preferred embodiment forward and reverse primers are designed on the basis of Illumina adapters (Illumina), comprising from 5' to 3':
1) an Illumina adapter sequence (IA): required for Illumina sequencing;
   - an Index sequencing primer/flow cell binding sequence: this region is required for flow cell binding, as well as annealing sequence for i5/i7 index sequencing primers;
   - i5/i7 indexes: indexes used for NGS multiplexing reaction;
   - a Read 1/read 2 sequencing primer: annealing sequence for Illumina sequencing primers, as well as for the amplification of the library from tagged oligos.
2) A first amplification handle sequence (1AH) or a sequence reverse complementary to the second amplification handle sequence (2AH-RC): these sequences anneal with the reverse complement of the first amplification handle sequence and with the second amplification handle sequence, respectively, on the tagged oligo, which are double stranded following tagged oligo amplification.

Figure 9 shows the structure of the library primers used for the generation of a massively parallel sequencing library from the amplified tagged oligonucleotides respectively in the embodiment according to Figure 5 (Figure 9A), Figure 6A (Figure 9B) and Figure 8 (Figure 9C).

After the generation of the library, at least one purification step is preferably performed, followed by library quantification and pooling necessary for the subsequent sequencing procedure. Sequencing is preferentially performed as paired-end sequencing and two reads, each deriving from a strand of the library DNA molecule, are generated.

The same approach can be used to produce NGS libraries for other sequencing platforms, such as for example Ion Torrent.

Analysis of the paired-end read sequences generated from the NGS libraries are analysed according to the following steps:
1. sub-sequences extraction. Sub-sequences corresponding to UMI, BAB and amplification handle sequence/s (1AH and/or 2AH) are extracted from both sequencing reads for each tagged oligo molecule.
2. Read re-alignment. In case sub-sequences of BAB and/or amplification handle sequence/s do not match reference sequences (with a tolerance of 0.5-2 mismatches every 5 bases), sub-sequences position is offset by a variable amount, ranging from -n to +n, where n is the maximum offset allowed (for example n=8) and sub-sequences are re-extracted. For each iteration the Hamming distance from reference sequences is calculated and the offset returning the lowest distance is selected and all sub-sequences (UMI, BAB, amplification handle sequences) are extracted.
3. Reads filtering. Reads the BAB and/or handle sub-sequences of which differ from reference sequences more than a defined amount of bases are discarded as low quality reads.
4. UMI determination. UMI sequences from read pairs are expected to be perfectly complementary in the absence of sequencing errors. In the presence of any differences between the first strand UMI and second strand complementary UMI sequences:
   a. the read pair may be discarded as low confidence or
   b. a consensus between the two sequences can be calculated by selecting, for each position of the UMI, the base, among the sequences from the two sequencing reads, which have the highest base calling score as reported by the sequencer base caller.
   It should be noted that the first method (a) is less prone to lead to an overestimation of target molecules due to sequencing biases but may lose true binding events between the binding agent and the target molecule, which are instead recovered with the second method (b).
5. Target molecules quantification. Counting of target molecules is performed by determining the number of distinct UMI sequences for each BAB sequence, representing a specific binding agent, in the analyzed sample.

A kit for carrying out the method according to the present invention comprises:
a) at least one binding agent directed to at least one target molecule in a biological sample conjugated with a tagged oligonucleotide, the tagged oligonucleotide comprising:
   i) a payload sequence of nucleic acid (PL) comprising a binding-agent barcode sequence (BAB) and a unique molecular identifier sequence (UMI),
   ii) at least one first tagged oligonucleotide amplification sequence (5-TOS);
b) at least one primer for carrying out a whole genome amplification and at least one primer for carrying out an amplification of the tagged oligonucleotide, the at least one primer for carrying out a whole genome amplification and at least one primer for carrying out an amplification of the tagged oligonucleotide having the same sequence.

In a preferred embodiment, the kit comprises an oligonucleotide for extending the tagged oligonucleotide.

In a preferred embodiment, the at least one tagged oligonucleotide has a sequence corresponding to SEQ ID NO:1, the primers for carrying out the amplification of the tagged oligonucleotide are two and have respectively sequence SEQ ID NO:2 and SEQ ID NO:3. The kit further preferably comprises one or more first library primer/s and one or more second library primer/s. More preferably, said first library primer/s has/have a sequence selected from the group consisting of SEQ ID NO:8 to SEQ ID NO:15 and said second library primer/s has/have a sequence selected from the group consisting of SEQ ID NO:16 to SEQ ID NO:27.

In another preferred embodiment, the at least one tagged oligonucleotide has a sequence corresponding to SEQ ID NO:28, and the oligonucleotide for carrying out the extension of the tagged oligonucleotide is one and has a sequence corresponding to SEQ ID NO:29. The kit preferably further comprises one or more first library primer/s and one or more second library primer/s. More preferably, said first library primer/s has/have a sequence selected from the group consisting of SEQ ID NO:30 to SEQ ID NO:37 and said second library primer/s has/have a sequence selected from the group consisting of SEQ ID NO:38 to SEQ ID NO:49.

### Examples

### Example 1

In this example tagged oligos were designed to perform amplification with the set-up according to Figure 8, after WGA. The tagged oligos, named "P5-Synth" (SEQ ID NO:1, Figure 10, NNNNNNNNNN: UMI sequence), were designed to be compatible with the Ampli1^{™} WGA kit (Menarini Silicon Biosystems). The first amplification handle sequence (1AH) was identical to the last 19 bases of Index 2 (i5) Adapters from Illumina TruSeq DNA and RNA CD Indexes. The second amplification handle sequence (2AH) was generated *in silico* so as to avoid any intramolecular secondary structures and possible matches on the human genome. The melting temperatures of both amplification handle sequences were designed in order to be similar to that of the WGA primer. Tagged oligo amplification primers (SEQ ID NO:2 and SEQ ID NO:3) were designed according to first and second amplification handle sequences.

As shown in Figure 10, the forward library primer was identical to Index 2 (i5) Adapters (Illumina), whereas the reverse library primer was identical to Index 1 (i7) Adapters with the addition of a reverse complementary sequence of second amplification handle sequence. More in detail, Figure 10 shows the design of P5-Synth oligo and corresponding library primers.

Oligo P5-Synth (SEQ ID NO:1): the white box indicates the internal domain with the UMI and Binding agent barcode. Grey boxes with black border indicate the first and second amplification handle sequences.

P5 library primer (SEQ ID NO:8): the forward primer used for NGS library generation. In the grey box the annealing site with Oligo P5-Synth. In the short dashed box the sequencing primer site; in dashed-dotted box the i5 index used for multiplexing sequencing reactions; in the long dashed box the index sequencing primer/flow cell adapter sequence.

Synth library primer (SEQ ID NO:16): the reverse primer used for NGS library generation. In the grey box the annealing site with Oligo P5-Synth. In the short dashed box the sequencing primer site; in dashed-dotted box the i5 index used for multiplexing sequencing reactions; in the long dashed box the index sequencing primer/flow cell adapter sequence.

As can be seen in Figure 11, during library generation the Illumina Index 1 and 2 Adapters are added to the 5' and 3' of the tagged oligo respectively.

In this example tagged oligos were conjugated via a 5' amino modifier. A C6 or C12 spacer was present between the amine moiety and the 5' of the oligo to avoid any steric hindrance inhibition effects on subsequent PCR reactions. Antibodies were covalently bound to tagged oligos using amines normally present in the antibody from lysine, glutamine, arginine and asparagine residues, through an amino-reactive reagent. Four Ab-oligos (Table 2) have been generated with the tagged oligos and antibody oligo conjugation was performed by Expedeon Ltd (25 Norman Way, Over, Cambridge CB24 5QE, United Kingdom) with an antibody:tagged oligo stoichiometry of 1:2. Epitope localization: indicates the position respect to the cell membrane.

**Table 2**

| **Name** | **Antibody** | **Isotype** | **Antibody name** | **Binding Agent Barcode** | **Epitope localization** |
|---|---|---|---|---|---|
| Ab-oligo tag1 | anti Pan-Cytokeratin | Mouse IgG1 | antiCK | TACTCATGCT (SEQ ID NO:4) | Internal |
| Ab-oligo tag2 | anti Her2 | Mouse IgG1 | antiHer2 | AGATAGCGCA (SEQ ID NO:5) | Internal |
| Ab-oligo tag3 | anti CD45 | Mouse IgG2a | antiCD45 | TCTCTCGCTG (SEQ ID NO:6) | External |
| Ab-oligo tag4 | IgG1 isotype control | Mouse IgG1 | IgGlisotype | CTGAGTCAGA (SEQ ID NO:7) | - |

Ab-oligos were used to stain two different types of cell lines. The first cell type was SK-BR-3 cells which are a breast tumor derived cell line which overexpresses cytokeratin and the Her2 protein. The second cell type was Peripheral Blood Mononuclear Cells (PBMCs) which are white blood cells extracted from whole blood which express CD45 and negligible levels of cytokeratin and Her2.

SK-BR-3 cells (ATCC^{®} HTB-30^{™}, ATCC) were grown in culture according to the manufacturer's procedure. PBMCs were extracted from human blood samples. Both cell types were fixed using PFA 2% according to a customised protocol.

Cell staining with Ab-oligos was performed on 100,000 - 50,000 previously fixed and permeabilised cells. Cells were collected by centrifugation at 1000 x g, for 5 minutes at RT. Cells were washed with at least 1 mL of Running Buffer (autoMACS Running Buffer, ref. 130-091-221, Miltenyi Biotec) and collected by centrifugation. This last step was repeated two times. External Ab-oligos and their isotype Ab-oligo controls are diluted in 100 *µ*l of Running Buffer down to their working concentration. External Ab-oligo mix (Ab-oligo tag3) was added to cells and incubated 15 minutes at RT. Sample was subsequently washed twice with 1 mL of Running Buffer and collected by centrifugation. Goat anti Mouse IgG2a - PE antibody in 500 *µ*l of Running Buffer was added and incubated for 30 minutes at +4°C. This step enabled the staining of PBMC cells in PE. The sample was washed twice with Running Buffer. Internal Ab-oligos and their isotype Ab-oligo controls are diluted in 200 *µ*l of Inside Perm Buffer (Inside Stain Kit, Ref. 130-090-477, Miltenyi Biotec) down to their working concentrations. Internal Ab-oligo mix (Ab-oligo tag1, 2 and 4) was added to cells and incubated for 10 minutes at RT. Sample was washed twice with 1 mL of Inside Perm Buffer and collected by centrifugation. A mix of Hoechst and Goat anti Mouse IgG1 - APC antibody in 500 *µ*l of Inside Perm Buffer was added and incubated for 30 minutes at +4°C. This step enabled the staining of SK-BR-3 cells in PE and all cell nuclei. The sample was washed twice with Running Buffer.

The addition of secondary antibodies conjugated to fluorophores enabled the identification of SK-BR-3 cells (APC channel) and PBMCs (PE channel) by fluorescence. Moreover the fluorescence levels reflect the relative abundance of Ab-oligo. Single-cells were purified using DEPArray^{™} NxT system (Menarini Silicon Biosystems) based on their immunofluorescent labeling (Figure 12).

Specifically, Figure 12 shows a scatterplot of the PBMC and SK-BR-3 cells stained with Ab-oligo and secondary fluorescent antibodies. On the x-axis the fluoresce levels in the APC channel, which is proportional to the quantity of Ab-oligo tag1, tag2 and tag4. On the y-axis the fluorescence levels in the PE channel, which is proportional to the quantity of Ab-oligo tag3. The scatterplot has been divided into four quadrants each containing a specific cell type based on their immunofluorescence levels: 1) PBMCs (high level of CD45 and low levels of CK,Her2); 2) double positive cells (high levels of CD45, CK, Her2); 3) double negative cells (low levels of CD45, CK, Her2); 4) SK-BR-3 cells (low level of CD45 and high levels of CK, Her2). Single cells highlighted with empty/filled square/circles were isolated and used for library generation.

Alternatively, to perform tagged oligo amplification after WGA a customized reaction mix of forward/reverse primers and Ampli1^{™} PCR kit reagents were prepared according to Table 3, left inset. Added 15 *µ*l of the reaction mix to each tube containing WGA products. Each sample was incubated according to thermal profile indicated in Table 3, right inset.

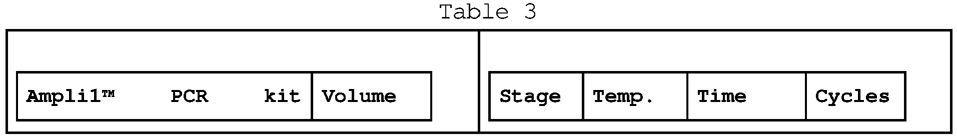

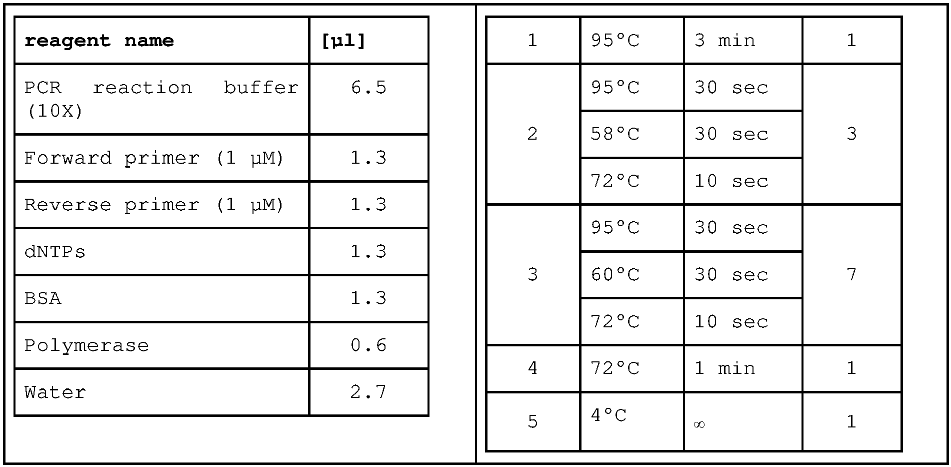

Left inset: reaction mixture composition of tagged oligo amplification reaction. Right inset: thermal cycling program for the tagged oligo amplification.

Library preparation was performed by taking an aliquot of 1 *µ*l of WGA containing Ab-oligo amplicons amplified using Ampli1^{™} PCR kit using P5 and Lib1 library primers at a final concentration of 0.5 *µ*M. PCR thermal cycling profile is indicated in Table 5. Each sample had a different combination of NGS library primers for dual indexing in order to demultiplex data during bioinformatic analysis. The list of the library primers used is reported in Table 4. P5 library primers are forward primers that can be used with tagged oligo P5-Synth.

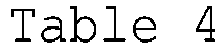

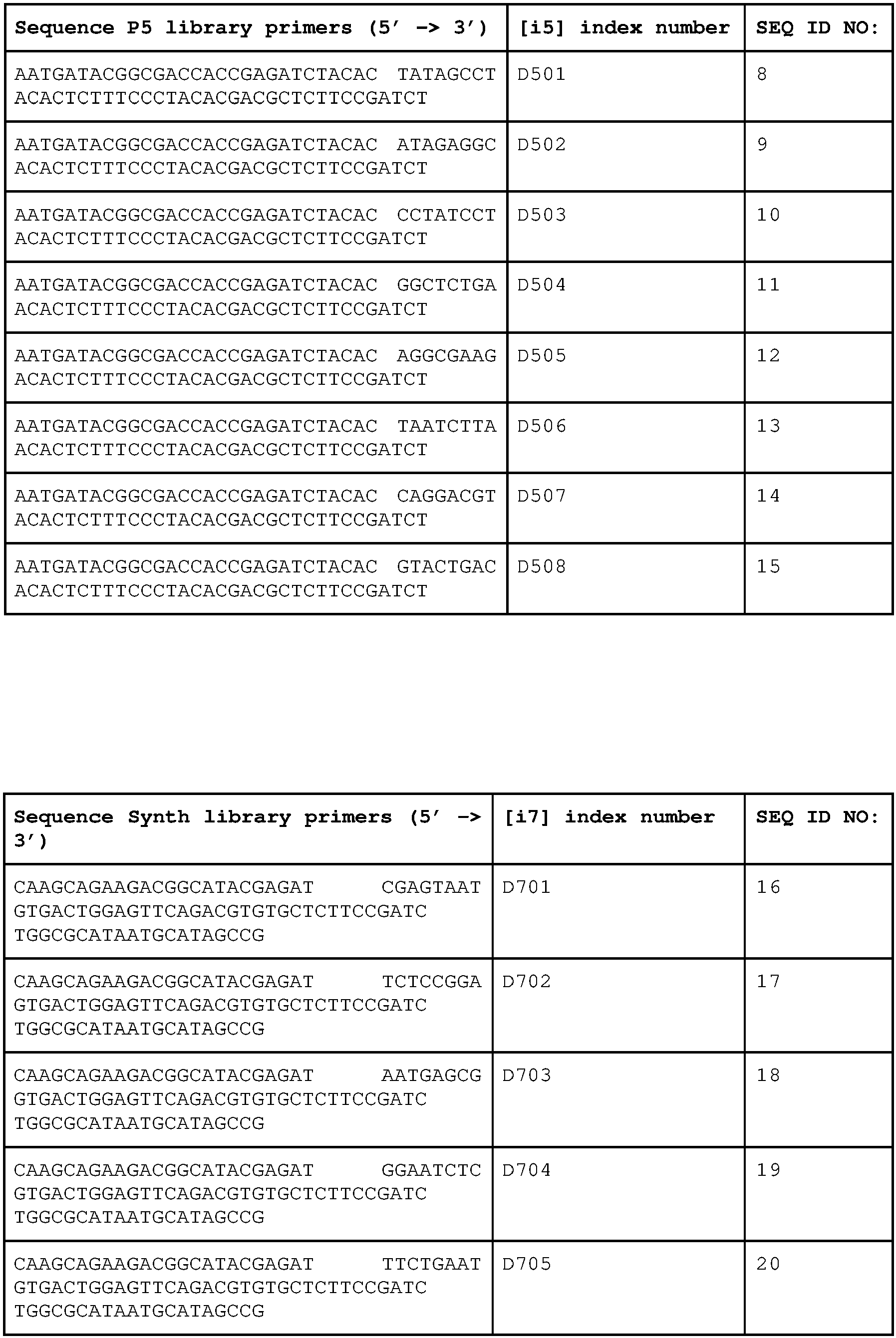

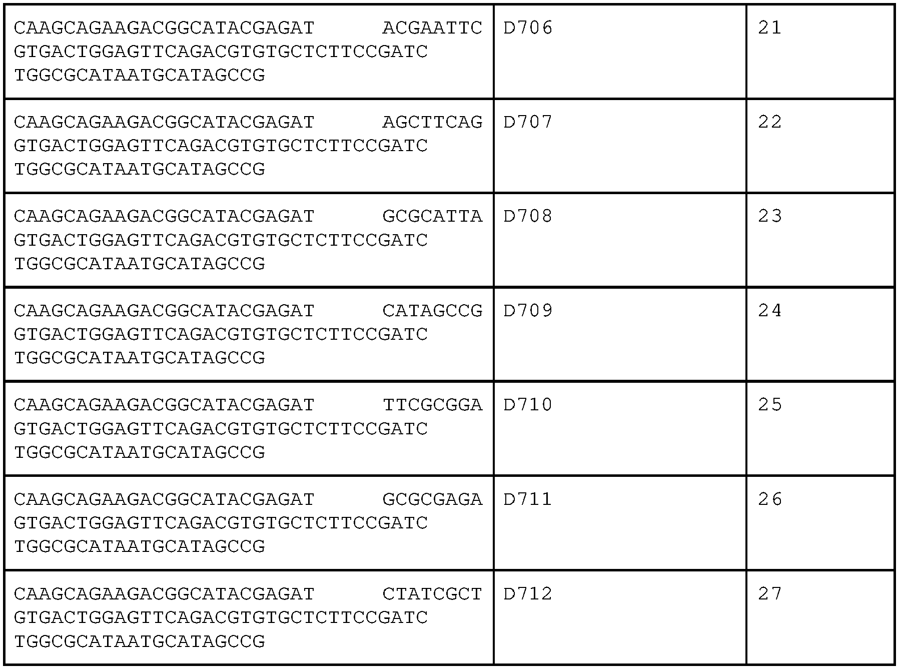

**Table 5**

| **Stage** | **Temperature** | **Time** | **Cycles** |
|---|---|---|---|
| 1 | 95°C | 3 min | 1 |
| 2 | 95°C | 30 sec | 3 |
| | 60°C | 30 sec | |
| | 72°C | 10 sec | |
| 3 | 95°C | 30 sec | 23-30 |
| | 65°C | 30 sec | |
| | 72°C | 10 sec | |
| 4 | 72°C | 1 min | 1 |
| 5 | 4°C | ∞ | 1 |

Thermal cycling profile for NGS library generation. The number of cycles during step 3 vary depending on the number of cells that have been recovered and the effective quantity of total Ab-oligos in cells. Usually 27 amplification cycles at stage 3 resulted in sufficient amplicon quantity from a single cell.

Library samples were purified using Agencourt AmPure XP beads (Beckman-Coulter). NGS DNA quantification was performed using KAPA SYBR^{®} FAST qPCR kit (Kapa Biosystems). Each NGS library was checked using Agilent Bioanalyzer 2100 (Agilent) showing a library electropherogram that was typically composed of a single peak at 185 bp (Figure 13).

Samples were pooled together and sequencing was performed on MiSeq system (Illumina) using MiSeq Reagent Kit v3 150-cycle (Ref. MS-102-3001, Illumina). Data analysis was performed using a custom software developed in Python. Quantification of protein targets according to UMIs counting is reported in Figure 14. As expected SK-BR-3 cells showed high expression of cytokeratin and Her2 and lower levels of CD45, while PBMC showed the opposite behaviour. Protein expression levels were high in double positive cells, especially isotype controls, indicating that such cells were more prone to non specific staining. Conversely double negative cells had lower levels for all four targets.

### Example 2

In this example tagged oligos were designed to perform amplification with the set-up according to Figure 8, during WGA. The experimental procedure is identical to Example 1 except for the following. Tagged oligo amplification primers were added directly to primary PCR reaction mix at a final concentration of 0.02 *µ*M. Library generation and data analysis were performed as indicated in Example 1.

Quantification of protein targets according to UMIs counting is reported in Figure 15. As expected SK-BR-3 cells showed high expression of cytokeratin and Her2 and very low levels of CD45, while PBMC showed the opposite behaviour. Protein expression levels were high in double positive cells, especially isotype controls, indicating that such cells were more prone to non specific staining. Conversely double negative cells had lower levels for all four targets. According to the results from Example 1 it can be inferred that the amplification of tagged oligo is feasible both during or after WGA. However, it has to be noted that the absolute UMI count differs significantly between the two procedures. Differences between the two cell types are more in line to what expected for CD45 target that has lower expression compared to CK, when tagged oligo amplification is performed during WGA.

### Example 3

In this example, tagged oligos were added directly in single cells. The tagged oligos, namely "P5-Lib1" (SEQ ID NO: 28), were designed to be amplifiable by Ampli1^{™} WGA kit (Menarini Silicon Biosystems). The tagged oligo amplification primer has sequence SEQ ID NO: 29 (forward and reverse primers are identical, sharing the sequence of Ampli1 WGA Lib1 primer). Specifically the 5'-WGA handle sequence was identical to the Lib1 WGA primer, while the 3'-WGA handle sequence was the reverse-complement sequence of the Lib1 WGA primer. The first amplification handle sequences are identical to those described in Example 1. The second amplification handle sequence consists of a 3'-WGA handle sequence and an additional 5 bp sequence at the 5' end of it (Figure 16).

More in detail, Figure 16 shows the design of P5-Synth oligo and corresponding library primers.

Oligo P5-Lib1: The white filled box with thick borders indicates the internal domain with the UMI and Binding agent barcode. Grey filled boxes with thick borders indicate the annealing sites for the two library primers. The grey boxes with thin borders are the WGA handle sequences (Lib1).

P5 library primer: the forward primer used for NGS library generation. In the grey filled box the annealing site with tagged oligo P5-Lib1. In the short dashed box the sequencing primer site; in dashed-dotted box the i5 index used for multiplexing sequencing reactions; in the long dashed box the index sequencing primer / flow cell adapter sequence.

Lib1 library primer: the reverse primer used for NGS library generation. In the grey filled box the annealing site with tagged oligo P5-Lib1: this sequence is composed of a part of Lib1 reverse complement sequence and a small tail (ACCAC) enabling annealing only to the 3' end of the Oligo P5-Lib1. In the short dashed box the sequencing primer site; in dashed-dotted box the i5 index used for multiplexing sequencing reactions; in the long dashed box the index sequencing primer / flow cell adapter sequence.

The forward library primer was identical to Index 2 (i5) Adapters (Illumina), whereas the reverse library primer was identical to Index 1 (i7) Adapters with the addition of a reverse complementary sequence of the second amplification handle sequence (Figure 16). Therefore, during library generation the Illumina Index 1 and 2 Adapters are added to the 5' and 3' of the tagged oligo respectively (Figure 17).

SK-BR-3 cells (ATCC^{®} HTB-30^{™}, ATCC) were grown in culture according to the manufacturer's procedure and were fixed using PFA 2% according to a customized protocol. Single-cells were purified using DEPArray^{™} NxT system (Menarini Silicon Biosystems) based on their morphology. P5-Lib1 and P5-Synth tagged oligos were added directly inside tubes containing single cells. Different quantities of each oligo were added within each single cell and performed Ampli1^{™} WGA. Samples containing P5-Synth tagged oligos were amplified as in Example 1.

Tagged oligos library generation was performed by taking an aliquot of 1 *µ*l of WGA containing Ab-oligo amplicons was amplified using Ampli1^{™} PCR kit using P5 and Lib1 library primers at a final concentration of 0.5 *µ*M . PCR thermal cycling profile is indicated in Table 5. Each sample had a different combination of NGS library primers for dual indexing in order to demultiplex data during bioinformatic analysis. The list of the library primers used is reported in Table 6.

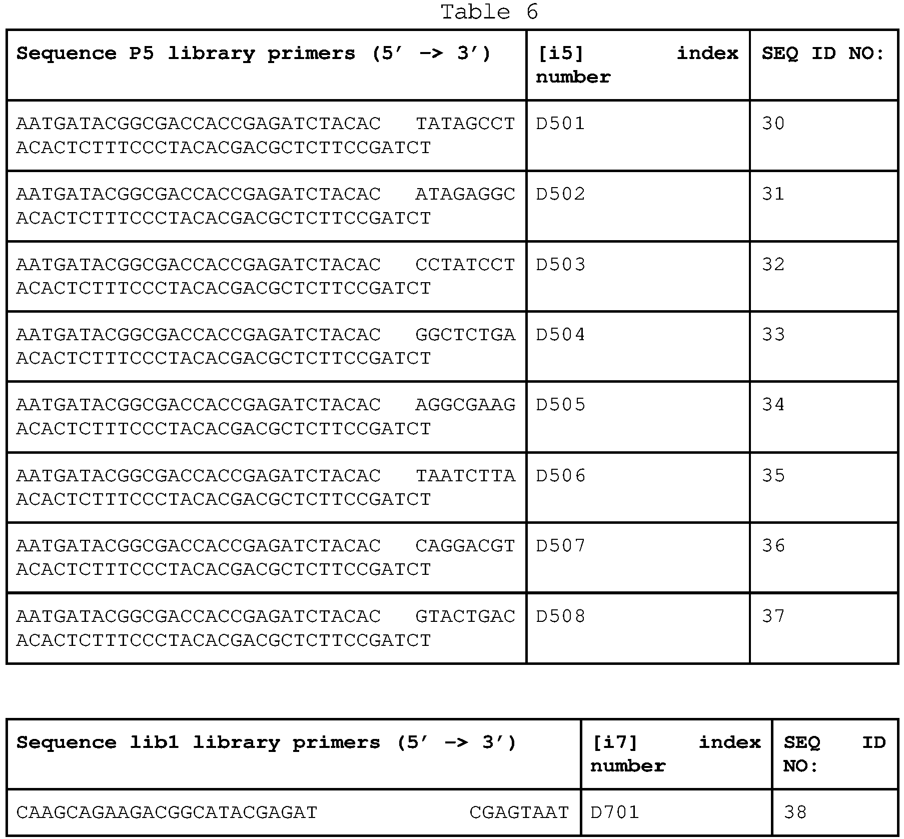

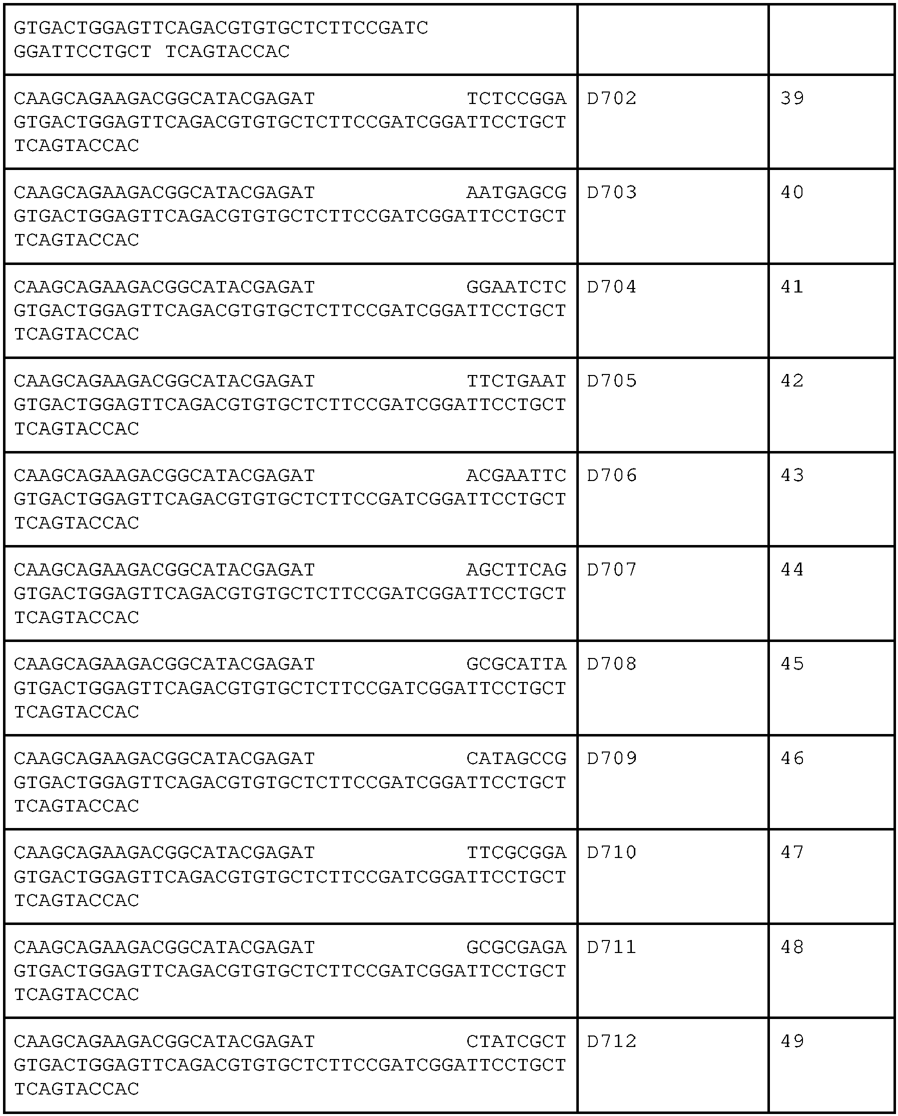

An aliquot of 10 *µ*l WGA samples were purified with SPRI beads (Beckmann Coulter) and subsequently processed with Ampli1^{™} LowPass kit to generate NGS libraries for CNA analysis. Spiking of tagged oligos in single cell before WGA procedure did not affect downstream genetic analyses (Figure 18). Tagged oligo that have been designed to fit workflows shown in Figure 5 and Figure 8 did not affect or interfere with the WGA procedure. Moreover, it was still possible to obtain NGS libraries from tagged oligonucleotides in both conditions. Both tagged oligonucleotides could be quantified correctly, indicating the robustness of both methodologies, as well as tagged oligo design (Figure 19).

### Advantages

The method for whole genome amplification and analysis of multiple target molecules in a biological sample according to the present invention allows to obtain simultaneously analysis of genome-wide copy-number profiles/genome sequence and protein expression on the same single cells.

The method of the present invention enables to perform whole genome amplification of genomic DNA, useful to enable further analysis such as genome-wide copy number profiling by low-pass sequencing or targeted sequencing of panels of genes of interest, and to detect and perform a digital quantification of a panel of multiple proteins down to single-cell resolution, using very few samples, as it may be the case where only few (down to a single one) circulating tumour cells (CTCs) are available. This is particularly advantageous over measuring each molecular type in different cells in a genetically heterogeneous sample where differences in genotype, phenotype and environment may confound and completely prevent the correlation of genotype (copy number of sequence alterations) with phenotype (protein expression).

The method according to the invention surprisingly advances the state of the art with performances previously thought unachievable by the skilled in the art, in one or more of the following dimensions, given by way of nonlimiting example:
- digital quantification of proteins in a single-cell down to hundreds of copies per cell.
- thanks to the use of an inherent WGA in the process, the above points are obtained with the further possibility to obtain additional genetic material for investigation of other characteristics of said single-cell, as well as the possibility to reliably reanalyse a single cell for verification, which is not possible with droplet-based approaches such as that proposed by 10x Genomics.

The primary field of application of the method is oncology, but the method can be applied to other fields such as mosaic disorders such as dermatological or overgrowth phenotypes.

## Claims

1. A method for whole genome amplification and analysis of multiple target molecules in a biological sample including genomic DNA and target molecules comprising the steps of:
a) providing the biological sample;
b) contacting the biological sample with at least one binding agent, which is directed to at least one of the target molecules, conjugated with a tagged oligonucleotide, the tagged oligonucleotide comprising:
i) a payload sequence of nucleic acid (PL) comprising a binding-agent barcode sequence (BAB) and a unique molecular identifier sequence (UMI), and
ii) at least one first tagged oligonucleotide amplification sequence of nucleic acid (5-TOS);
so that - when at least one target molecule is present in the biological sample - the at least one binding agent binds to the at least one target molecule;
c) carrying out a separating step to selectively remove unbound binding agent thus obtaining a labeled biological sample;
d) carrying out on the labeled biological sample:
- a whole genome amplification of said genomic DNA by:
i) deterministic restriction-site whole genome amplification (DRS-WGA), or
ii) Multiple Annealing and Looping Based Amplification Cycles whole genome amplification (MALBAC), and
- an amplification of the tagged oligonucleotide conjugated with the at least one binding agent,
wherein whole genome amplification and amplification of the tagged oligonucleotide are carried out simultaneously;
e) preparing a massively parallel sequencing library from the amplified tagged oligonucleotide;
f) sequencing the massively parallel sequencing library;
g) retrieving the sequences of the binding-agent barcode sequence (BAB) and unique molecular identifier sequence (UMI) from each sequencing read;
h) counting the number of distinct unique molecular identifier sequences (UMI) for each binding agent.

2. The method according to claim 1, wherein the tagged oligonucleotide further comprises at least one second tagged oligonucleotide amplification sequence (3-TOS).

3. The method according to claim 1 or 2, wherein said unique molecular identifier sequence (UMI) is a degenerate or semi-degenerate sequence in the range from 10 to 30 nucleotides.

4. The method according to any of the preceding claims, wherein the method further comprises a step of isolating a single cell from the biological sample.

5. The method according to claim 4, wherein said step of isolating is performed by sorting cells.

6. The method according to claim 4, wherein said step of isolating is performed by partitioning cells into droplets.

7. The method according to any of claims 4 to 6, wherein said step of isolating is performed after step c) and before step d).

8. The method according to any of the preceding claims, further comprising a step of purifying the massively parallel sequencing library before step f).

9. The method according to any of the preceding claims, wherein the at least one binding agent is selected from the group consisting of:
a) an antibody or fragment thereof,
b) an aptamer,
c) a small molecule,
d) a peptide, and
e) a protein.

10. The method according to any of the preceding claims, wherein the target molecule is selected from the group consisting of:
a) a protein,
b) a peptide,
c) a glycoprotein,
d) a carbohydrate,
e) a lipid, and
f) a combination thereof.

11. The method according to any of claims 2 to 10, wherein the tagged oligonucleotide comprises from 5' to 3' at least:
a) the first tagged oligonucleotide amplification sequence of nucleic acid (5-TOS), comprising in turn a 5' whole genome amplification handle sequence (5-WGAH) and a first amplification handle sequence (1AH);
b) the payload sequence (PL);
c) the second tagged oligonucleotide amplification sequence of nucleic acid (3-TOS), comprising in turn a second amplification handle sequence of nucleic acid (2AH) and a 3' whole genome amplification handle sequence (3-WGAH).

12. The method according to any of the preceding claims, wherein the whole genome amplification and the amplification of the tagged oligo are carried out using a single primer.

13. The method according to any of claims 1 to 10, wherein the tagged oligonucleotide comprises from 5' to 3' at least:
a) the first tagged oligonucleotide amplification sequence of nucleic acid (5-TOS), comprising in turn a 5' whole genome amplification handle sequence (5-WGAH) and a first amplification handle sequence (1AH);
b) the payload sequence (PL);
c) optionally, an annealing sequence (AS); and wherein at least one primer is used for whole genome amplification and amplification of the tagged oligonucleotide and at least one oligonucleotide (E-p) is used
for the extension of the tagged oligonucleotide, said at least one oligonucleotide (E-p) comprising from 5' to 3' at least:
d) the 5' whole genome amplification handle sequence (5-WGAH);
e) a spacer sequence (SS);
f) a second amplification handle sequence (2AH); and
g) a sequence reverse complementary to the annealing sequence (AS-RC) or a sequence reverse complementary to the binding-agent barcode sequence (BAB-RC).

14. The method according to any of claims 1 to 10, wherein the tagged oligonucleotide comprises from 5' to 3' at least:
a) the first tagged oligonucleotide amplification sequence of nucleic acid (5-TOS) corresponding to a first amplification handle sequence (1AH);
b) the payload sequence (PL);
c) the second tagged oligonucleotide amplification sequence of nucleic acid (3-TOS) corresponding to a second amplification handle sequence (2AH); and
wherein at least one first primer is used for whole genome amplification and at least one second and one third primer are used for the amplification of the tagged oligonucleotides;
the at least one second primer having a sequence identical to the first amplification handle sequence (1AH) and the at least one third primer having a sequence reverse complementary to the second amplification handle sequence (2AH-RC).

15. The method according to claim 14, wherein the at least one second primer and at least one third primer are added in step d).

16. A method according to any of claims 11 to 15, wherein said step e) of preparing a massively parallel sequencing library from the amplified tagged oligonucleotide is performed by a PCR reaction using at least one first library primer, comprising a 3' sequence corresponding to the first amplification handle sequence (1AH), and at least one second library primer comprising a 3' sequence corresponding to the sequence reverse complementary to the second amplification handle sequence (2AH-RC).

17. A kit for carrying out the method of any of claims 1 to 16 comprising:
a) at least one binding agent directed to at least one target molecule in a biological sample conjugated with a tagged oligonucleotide, the tagged oligonucleotide comprising:
i) a payload sequence of nucleic acid (PL) comprising a binding-agent barcode sequence (BAB) and a unique molecular identifier sequence (UMI),
ii) at least one first tagged oligonucleotide amplification sequence (5-TOS);
b) at least one primer for carrying out a whole genome amplification and at least one primer for carrying out an amplification of the tagged oligonucleotide, the at least one primer for carrying out a whole genome amplification and at least one primer for carrying out an amplification of the tagged oligonucleotide having the same sequence.

18. A kit according to claim 20, further comprising an oligonucleotide for extending the tagged oligonucleotide.

19. A kit according to claim 17 or 18, wherein the at least one tagged oligonucleotide has a sequence corresponding to SEQ ID NO:1, the primers for carrying out the amplification of the tagged oligonucleotide are two and have respectively sequence SEQ ID NO:2 and SEQ ID NO:3.

20. A kit according to any of claims 17 to 19, further comprising one or more first library primer/s and one or more second library primer/s.

21. A kit according to claim 20, wherein said first library primer/s has/have a sequence selected from the group consisting of SEQ ID NO:8 to SEQ ID NO:15 and said second library primer/s has/have a sequence selected from the group consisting of SEQ ID NO:16 to SEQ ID NO:27.

22. A kit according to claim 17 or 18, wherein the at least one tagged oligonucleotide has a sequence corresponding to SEQ ID NO:28, and the primer for carrying out the amplification of the tagged oligonucleotide is one and has a sequence corresponding to SEQ ID NO:29.

23. The kit according to claim 22, further comprising one or more first library primer/s and one or more second library primer/s.

24. A kit according to claim 23, wherein said first library primer/s has/have a sequence selected from the group consisting of SEQ ID NO:30 to SEQ ID NO:37 and said second library primer/s has/have a sequence selected from the group consisting of SEQ ID NO:38 to SEQ ID NO:49.

## Patentansprüche

1. Verfahren zur Gesamtgenomamplifikation und Analyse mehrerer Zielmoleküle in einer biologischen Probe, die genomische DNA und Zielmoleküle enthält, umfassend die Schritte:
a) Bereitstellen der biologischen Probe;
b) Inkontaktbringen der biologischen Probe mit mindestens einem Bindemittel, das auf mindestens eines der Zielmoleküle gerichtet ist, konjugiert mit einem markierten Oligonukleotid, wobei das markierte Oligonukleotid umfasst:
i) eine Payload-Sequenz einer Nukleinsäure (PL), die eine Bindemittel-Barcode-Sequenz (BAB) und eine eindeutige molekulare Identifikator-Sequenz (UMI) umfasst, und
ii) mindestens eine erste markierte Oligonukleotid-Amplifikationssequenz einer Nukleinsäure (5-TOS), so dass - wenn mindestens ein Zielmolekül in der biologischen Probe vorhanden ist - das mindestens eine Bindemittel an das mindestens eine Zielmolekül bindet;
c) Durchführen eines Trennschritts zum selektiven Entfernen von ungebundenem Bindemittel, wodurch eine markierte biologische Probe erhalten wird;
d) Durchführen an der markierten biologischen Probe:
- eine Gesamtgenomamplifikation der genomischen DNA durch:
i) deterministische Restriktionsstellen-Gesamtgenomamplifikation (DRS-WGA) oder
ii) Multiple Annealing and Looping Based Amplification Cycles Gesamtgenomamplifikation (MALBAC) und
- eine Amplifikation des markierten Oligonukleotids, das mit dem mindestens einen Bindemittel konjugiert ist, wobei die Gesamtgenomamplifikation und die Amplifikation des markierten Oligonukleotids gleichzeitig durchgeführt werden;
e) Herstellen einer massiv parallelen Sequenzierungsbibliothek aus dem amplifizierten markierten Oligonukleotid;
f) Sequenzieren der massiv parallelen Sequenzierungsbibliothek;
g) Abrufen der Sequenzen der Bindemittel-Barcode-Sequenz (BAB) und der eindeutigen molekularen Identifikator-Sequenz (UMI) aus jedem Sequenzierungslesevorgang;
h) Zählen der Anzahl unterschiedlicher eindeutiger molekularer Identifikator-Sequenzen (UMI) für jedes Bindemittel.

2. Verfahren nach Anspruch 1, wobei das markierte Oligonukleotid ferner mindestens eine zweite markierte Oligonukleotid-Amplifikationssequenz (3-TOS) umfasst.

3. Verfahren nach Anspruch 1 oder 2, wobei die eindeutige molekulare Identifikator-Sequenz (UMI) eine degenerierte oder halb degenerierte Sequenz im Bereich von 10 bis 30 Nukleotiden ist.

4. Verfahren nach einem der vorhergehenden Ansprüche, wobei das Verfahren ferner einen Schritt des Isolierens einer einzelnen Zelle aus der biologischen Probe umfasst.

5. Verfahren nach Anspruch 4, wobei der Schritt des Isolierens durch Sortieren von Zellen durchgeführt wird.

6. Verfahren nach Anspruch 4, wobei der Schritt des Isolierens durchgeführt wird, indem Zellen in Tröpfchen aufgeteilt werden.

7. Verfahren nach einem der Ansprüche 4 bis 6, wobei der Schritt des Isolierens nach Schritt c) und vor Schritt d) durchgeführt wird.

8. Verfahren nach einem der vorhergehenden Ansprüche, ferner umfassend einen Schritt des Aufreinigens der massiv parallelen Sequenzierungsbibliothek vor Schritt f).

9. Verfahren nach einem der vorhergehenden Ansprüche, wobei das mindestens eine Bindemittel ausgewählt ist aus der Gruppe bestehend aus:
a) einem Antikörper oder Fragment davon,
b) einem Aptamer,
c) einer niedermolekularen Verbindung,
d) einem Peptid, und
e) einem Protein.

10. Verfahren nach einem der vorhergehenden Ansprüche, wobei das Zielmolekül ausgewählt ist aus der Gruppe bestehend aus:
a) einem Protein,
b) einem Peptid,
c) einem Glykoprotein,
d) einem Kohlenhydrat,
e) einem Lipid und
f) einer Kombination davon.

11. Verfahren nach einem der Ansprüche 2 bis 10, wobei das markierte Oligonukleotid von 5' bis 3' mindestens umfasst:
a) die erste markierte Oligonukleotid-Amplifikationssequenz einer Nukleinsäure (5-TOS), die wiederum eine 5'-Gesamtgenomamplifikations-Handle-Sequenz (5-WGAH) und eine erste Amplifikations-Handle-Sequenz (1AH) umfasst;
b) die Payload-Sequenz (PL);
c) die zweite markierte Oligonukleotid-Amplifikationssequenz einer Nukleinsäure (3-TOS), die wiederum eine zweite Amplifikations-Handle-Sequenz einer Nukleinsäure (2AH) und eine 3'- Gesamtgenomamplifikations-Handle-Sequenz (3-WGAH) umfasst.

12. Verfahren nach einem der vorhergehenden Ansprüche, wobei die Gesamtgenomamplifikation und die Amplifikation des markierten Oligonukleotids unter Verwendung eines einzigen Primers durchgeführt werden.

13. Verfahren nach einem der Ansprüche 1 bis 10, wobei das markierte Oligonukleotid von 5' bis 3' mindestens umfasst:
a) die erste markierte Oligonukleotid-Amplifikationssequenz einer Nukleinsäure (5-TOS), die wiederum eine 5'-Gesamtgenomamplifikations-Handle-Sequenz (5-WGAH) und eine erste Amplifikations-Handle-Sequenz (1AH) umfasst;
b) die Payload-Sequenz (PL);
c) gegebenenfalls eine Annealing-Sequenz (AS); und
wobei mindestens ein Primer für die Gesamtgenomamplifikation und die Amplifikation des markierten Oligonukleotids verwendet wird und mindestens ein Oligonukleotid (E-p) verwendet wird
für die Verlängerung des markierten Oligonukleotids, wobei das mindestens eine Oligonukleotid (E-p) von 5' bis 3 mindestens umfasst:
d) die 5'-Gesamtgenomamplifikations-Handle-Sequenz (5-WGAH);
e) eine Spacer-Sequenz (SS);
f) eine zweite Amplifikations-Handle-Sequenz (2AH); und
g) eine zu der Annealing-Sequenz umgekehrt komplementäre Sequenz (AS-RC) oder eine zu der Bindemittel-Barcode-Sequenz umgekehrt komplementäre Sequenz (BAB-RC).

14. Verfahren nach einem der Ansprüche 1 bis 10, wobei das markierte Oligonukleotid von 5' bis 3' mindestens umfasst:
a) die erste markierte Oligonukleotid-Amplifikationssequenz einer Nukleinsäure (5-TOS), die einer ersten Amplifikations-Handle-Sequenz (1AH) entspricht;
b) die Payload-Sequenz (PL);
c) die zweite markierte Oligonukleotid-Amplifikationssequenz einer Nukleinsäure (3-TOS), die einer zweiten Amplifikations-Handle-Sequenz (2AH) entspricht; und
wobei mindestens ein erster Primer für die Gesamtgenomamplifikation verwendet wird und mindestens ein zweiter und ein dritter Primer für die Amplifikation der markierten Oligonukleotide verwendet werden;
wobei der mindestens eine zweite Primer eine zur ersten Amplifikations-Handle-Sequenz (1AH) identische Sequenz aufweist und der mindestens eine dritte Primer eine zur zweiten Amplifikations-Handle-Sequenz umgekehrt komplementäre Sequenz (2AH-RC) aufweist.

15. Verfahren nach Anspruch 14, wobei der mindestens eine zweite Primer und der mindestens ein dritter Primer in Schritt d) zugegeben werden.

16. Verfahren nach einem der Ansprüche 11 bis 15, wobei der Schritt e) des Herstellens einer massiv parallelen Sequenzierungsbibliothek aus dem amplifizierten markierten Oligonukleotid durch eine PCR-Reaktion unter Verwendung mindestens eines Primers einer ersten Bibliothek durchgeführt wird, der eine 3'-Sequenz umfasst, die der ersten Amplifikations-Handle-Sequenz (1AH) entspricht, und mindestens eines Primers einer zweiten Bibliothek, der eine 3'-Sequenz umfasst, die der Sequenz entspricht, die umgekehrt komplementär zu der zweiten Amplifikations-Handle-Sequenz (2AH-RC) ist.

17. Kit zur Durchführung des Verfahrens nach einem der Ansprüche 1 bis 16 umfassend:
a) mindestens ein Bindemittel, gerichtet auf mindestens ein Zielmolekül in einer biologischen Probe, konjugiert mit einem markierten Oligonukleotid, wobei das markierte Oligonukleotid umfasst:
i) eine Payload-Sequenz einer Nukleinsäure (PL), die eine Bindemittel-Barcode-Sequenz (BAB) und eine eindeutige molekulare Identifikator-Sequenz (UMI) umfasst, und
ii) mindestens eine erste markierte Oligonukleotid-Amplifikationssequenz (5-TOS),
b) mindestens einen Primer zur Durchführung einer Gesamtgenom-Amplifikation und mindestens einen Primer zur Durchführung einer Amplifikation des markierten Oligonukleotids, wobei der mindestens eine Primer zur Durchführung einer Gesamtgenom-Amplifikation und der mindestens eine Primer zur Durchführung einer Amplifikation des markierten Oligonukleotids, dieselbe Sequenz aufweisen.

18. Kit nach Anspruch 20, ferner umfassend ein Oligonukleotid zum Verlängern des markierten Oligonukleotids.

19. Kit nach Anspruch 17 oder 18, wobei das mindestens eine markierte Oligonukleotid eine Sequenz entsprechend der SEQ ID NO:1 aufweist, die Primer zum Durchführen der Amplifikation des markierten Oligonukleotids zwei sind und die Sequenz der SEQ ID NO:2 bzw. der SEQ ID NO:3 aufweisen.

20. Kit nach einem der Ansprüche 17 bis 19, ferner umfassend einen oder mehrere Primer einer ersten Bibliothek und einen oder mehrere Primer einer zweiten Bibliothek.

21. Kit nach Anspruch 20, wobei der/die Primer einer ersten Bibliothek eine Sequenz aufweist/aufweisen, ausgewählt aus der Gruppe bestehend aus SEQ ID NO:8 bis SEQ ID NO:15, und der/die Primer einer zweiten Bibliothek eine Sequenz aufweist/aufweisen, ausgewählt aus der Gruppe bestehend aus SEQ ID NO: 16 bis SEQ ID NO:27.

22. Kit nach Anspruch 17 oder 18, wobei das mindestens eine markierte Oligonukleotid eine Sequenz aufweist, die der SEQ ID NO: 28 entspricht, und der Primer zum Durchführen der Amplifikation des markierten Oligonukleotids einer ist und eine Sequenz aufweist, die der SEQ ID NO: 29 entspricht.

23. Kit nach Anspruch 22, ferner umfassend einen oder mehrere Primer einer ersten Bibliothek und einen oder mehrere Primer einer zweiten Bibliothek.

24. Kit nach Anspruch 23, wobei der/die Primer der ersten Bibliothek eine Sequenz aufweist/aufweisen, ausgewählt aus der Gruppe bestehend aus SEQ ID NO:30 bis SEQ ID NO:37, und der/die Primer der zweiten Bibliothek eine Sequenz aufweist/aufweisen, ausgewählt aus der Gruppe bestehend aus SEQ ID NO:38 bis SEQ ID NO:49.

## Revendications

1. Procédé d'amplification du génome entier et d'analyse de plusieurs molécules cibles dans un échantillon biologique incluant de l'ADN génomique et des molécules cibles comprenant les étapes de :
a) fourniture de l'échantillon biologique ;
b) mise en contact de l'échantillon biologique avec au moins un agent liant, qui cible au moins l'une des molécules cibles, conjugué à un oligonucléotide marqué, l'oligonucléotide marqué comprenant :
i) une séquence de charge utile d'acide nucléique (PL) comprenant une séquence de code-barres d'agent liant (BAB) et une séquence d'identifiant moléculaire unique (UMI), et
ii) au moins une première séquence d'amplification d'oligonucléotide marqué de l'acide nucléique (5-TOS) ;
de sorte que, lorsqu'au moins une molécule cible est présente dans l'échantillon biologique, l'au moins un agent liant se lie à au moins une molécule cible ;
c) réalisation d'une étape de séparation pour éliminer de manière sélective l'agent liant non lié, obtenant ainsi un échantillon biologique étiqueté ;
d) réalisation sur l'échantillon biologique étiqueté :
- d'une amplification du génome entier dudit ADN génomique par :
i) une amplification du génome entier par site de restriction déterministe (DRS-WGA), ou
ii) une amplification du génome entier par de multiples cycles d'amplification basés sur l'annelage et le bouclage (MALBAC), et
- d'une amplification de l'oligonucléotide marqué conjugué à l'au moins un agent liant,
dans lequel l'amplification du génome entier et l'amplification de l'oligonucléotide marqué sont réalisées simultanément ;
e) préparation d'une bibliothèque de séquençage massivement parallèle à partir de l'oligonucléotide marqué amplifié ;
f) séquençage de la bibliothèque de séquençage massivement parallèle ;
g) récupération des séquences de la séquence de code-barre d'agent liant (BAB) et de la séquence d'identifiant moléculaire unique (UMI) à partir de chaque lecture de séquençage ;
h) comptage du nombre de séquences distinctes d'identifiant moléculaire unique (UMI) pour chaque agent liant.

2. Procédé selon la revendication 1, dans lequel l'oligonucléotide marqué comprend en outre au moins une seconde séquence d'amplification d'oligonucléotide marqué (3-TOS).

3. Procédé selon la revendication 1 ou 2, dans lequel ladite séquence d'identifiant moléculaire unique (UMI) est une séquence dégénérée ou semi-dégénérée dans la plage de 10 à 30 nucléotides.

4. Procédé selon l'une quelconque des revendications précédentes, dans lequel le procédé comprend en outre une étape d'isolement d'une seule cellule de l'échantillon biologique.

5. Procédé selon la revendication 4, dans lequel ladite étape d'isolement est effectuée par tri de cellules.

6. Procédé selon la revendication 4, dans lequel ladite étape d'isolement est effectuée par cloisonnement de cellules en gouttelettes.

7. Procédé selon l'une quelconque des revendications 4 à 6, dans lequel ladite étape d'isolement est effectuée après l'étape c) et avant l'étape d) .

8. Procédé selon l'une quelconque des revendications précédentes, comprenant en outre une étape de purification de la bibliothèque de séquençage massivement parallèle avant l'étape f).

9. Procédé selon l'une quelconque des revendications précédentes, dans lequel l'au moins un agent liant est choisi dans le groupe consistant en :
a) un anticorps ou fragment de celui-ci,
b) un aptamère,
c) une petite molécule,
d) un peptide, et
e) une protéine.

10. Procédé selon l'une quelconque des revendications précédentes, dans lequel la molécule cible est choisie dans le groupe consistant en :
a) une protéine,
b) un peptide,
c) une glycoprotéine.
d) un glucide,
e) un lipide, et
f) une combinaison de ceux-ci.

11. Procédé selon l'une quelconque des revendications 2 à 10, dans lequel l'oligonucléotide marqué comprend de 5' à 3' au moins :
a) la première séquence d'amplification d'oligonucléotide marqué de l'acide nucléique (5-TOS), comprenant à son tour une séquence de manipulation d'amplification du génome entier 5' (5-WGAH) et une première séquence de manipulation d'amplification (1AH) ;
b) la séquence de charge utile (PL) ;
c) la seconde séquence d'amplification d'oligonucléotide marqué de l'acide nucléique (3-TOS), comprenant à son tour une seconde séquence de manipulation d'amplification de l'acide nucléique (2AH) et une séquence de manipulation d'amplification du génome entier 3' (3-WGAH).

12. Procédé selon l'une quelconque des revendications précédentes, dans lequel l'amplification du génome entier et l'amplification de l'oligo marqué sont réalisées à l'aide d'une seule amorce.

13. Procédé selon l'une quelconque des revendications 1 à 10, dans lequel l'oligonucléotide marqué comprend de 5' à 3' au moins :
a) la première séquence d'amplification d'oligonucléotide marqué de l'acide nucléique (5-TOS), comprenant à son tour une séquence de manipulation d'amplification du génome entier 5' (5-WGAH) et une première séquence de manipulation d'amplification (1AH) ;
b) la séquence de charge utile (PL) ;
c) facultativement, une séquence d'annelage (AS) ; et
dans lequel au moins une amorce est utilisée pour l'amplification du génome entier et l'amplification de l'oligonucléotide marqué et au moins un oligonucléotide (E-p) est utilisé
pour l'extension de l'oligonucléotide marqué, ledit au moins un oligonucléotide (E-p) comprenant de 5' à 3' au moins :
d) la séquence de manipulation d'amplification du génome entier 5' (5-WGAH) ;
e) une séquence d'espacement (SS) ;
f) une seconde séquence de manipulation d'amplification (2AH) ; et
g) une séquence inverse complémentaire de la séquence d'annelage (AS-RC) ou une séquence inverse complémentaire de la séquence de code-barre d'agent liant (BAB-RC).

14. Procédé selon l'une quelconque des revendications 1 à 10, dans lequel l'oligonucléotide marqué comprend de 5' à 3' au moins :
a) la première séquence d'amplification d'oligonucléotide marqué de l'acide nucléique (5-TOS) correspondant à une première séquence de manipulation d'amplification (1AH) ;
b) la séquence de charge utile (PL) ;
c) la seconde séquence d'amplification d'oligonucléotide marqué de l'acide nucléique (3-TOS) correspondant à une seconde séquence de manipulation d'amplification (2AH) ; et
dans lequel au moins une première amorce est utilisée pour l'amplification du génome entier et au moins une deuxième et une troisième amorces sont utilisées pour l'amplification des oligonucléotides marqués ;
l'au moins une deuxième amorce ayant une séquence identique à la première séquence de manipulation d'amplification (1AH) et l'au moins une troisième amorce ayant une séquence inverse complémentaire de la seconde séquence de manipulation d'amplification (2AH-RC).

15. Procédé selon la revendication 14, dans lequel l'au moins une deuxième amorce et l'au moins une troisième amorce sont ajoutées à l'étape d).

16. Procédé selon l'une quelconque des revendications 11 à 15, dans lequel ladite étape e) de préparation d'une bibliothèque de séquençage massivement parallèle à partir de l'oligonucléotide marqué amplifié est effectuée par une réaction PCR à l'aide d'au moins une première amorce de bibliothèque, comprenant une séquence 3' correspondant à la première séquence de manipulation d'amplification (1AH), et d'au moins une deuxième amorce de bibliothèque comprenant une séquence 3' correspondant à la séquence inverse complémentaire de la seconde séquence de manipulation d'amplification (2AH-RC).

17. Kit de réalisation du procédé de l'une quelconque des revendications 1 à 16 comprenant :
a) au moins un agent liant ciblant au moins une molécule cible dans un échantillon biologique conjugué à un oligonucléotide marqué, l'oligonucléotide marqué comprenant :
i) une séquence de charge utile de l'acide nucléique (PL) comprenant une séquence de code-barre d'agent liant (BAB) et une séquence d'identifiant moléculaire unique (UMI),
ii) au moins une première séquence d'amplification d'oligonucléotide marqué (5-TOS) ;
b) au moins une amorce pour la réalisation d'une amplification du génome entier et au moins une amorce pour la réalisation d'une amplification de l'oligonucléotide marqué, au moins une amorce pour la réalisation d'une amplification du génome entier et au moins une amorce pour la réalisation d'une amplification de l'oligonucléotide marqué ayant la même séquence.

18. Kit selon la revendication 20, comprenant en outre un oligonucléotide pour l'extension de l'oligonucléotide marqué.

19. Kit selon la revendication 17 ou 18, dans lequel l'au moins un oligonucléotide marqué a une séquence correspondant à SEQ ID NO:1, les amorces pour la réalisation de l'amplification de l'oligonucléotide marqué sont au nombre de deux et ont respectivement la séquence SEQ ID NO:2 et SEQ ID NO:3.

20. Kit selon l'une quelconque des revendications 17 à 19, comprenant en outre une ou plusieurs premières amorces de bibliothèque et une ou plusieurs deuxièmes amorces de bibliothèque.

21. Kit selon la revendication 20, dans lequel ladite ou lesdites premières amorces de bibliothèque ont une séquence choisie dans le groupe consistant en SEQ ID NO: 8 à SEQ ID NO: 15 et ladite ou lesdites deuxièmes amorces de bibliothèque ont une séquence choisie dans le groupe consistant en SEQ ID NO:16 à SEQ ID NO:27.

22. Kit selon la revendication 17 ou 18, dans lequel l'au moins un oligonucléotide marqué a une séquence correspondant à SEQ ID NO:28, et l'amorce pour la réalisation de l'amplification de l'oligonucléotide marqué est au nombre d'un et a une séquence correspondant à SEQ ID NO:29.

23. Kit selon la revendication 22, comprenant en outre une ou plusieurs premières amorces de bibliothèque et une ou plusieurs deuxièmes amorces de bibliothèque.

24. Kit selon la revendication 23, dans lequel ladite ou lesdites premières amorces de bibliothèque ont une séquence choisie dans le groupe consistant en SEQ ID NO:30 à SEQ ID NO:37 et ladite ou lesdites deuxièmes amorces de bibliothèque ont une séquence choisie dans le groupe consistant en SEQ ID NO:38 à SEQ ID NO:49.
